# EUROPÄISCHE PATENTSCHRIFT

(11) **EP 2 037 883 B1**
(45) Veröffentlichungstag und Bekanntmachung des Hinweises auf die Patenterteilung: **06.01.2010**
(21) Anmeldenummer: 07730313.9
(22) Anmeldetag: 28.06.2007
(51) Int. Cl.: A61K 9/14, A61K 9/16, A61K 31/155, A61K 47/42, B01D 1/18, B01J 2/04

(54) **INHALATIVE PULVER ENTHALTEND PHENYLALANIN**
INHALANT POWDER CONTAINING PHENYLALANINE
POUDRE À INHALER COMPRENNANT PHENYLALANINE

(30) Priorität: 29.06.2006 DE 102006030164
(43) Veröffentlichungstag der Anmeldung: 25.03.2009
(73) Patentinhaber: Boehringer Ingelheim Pharma GmbH & Co. KG, 55216 Ingelheim am Rhein (DE)
(72) Erfinder: SCHULTZ-FADEMRECHT, Torsten, 88437 Maselheim (DE); GARIDEL, Patrick, 22846 Norderstedt (DE); BECHTOLD-PETERS, Karoline, 88400 Biberach (DE); FISCHER, Beate, 88400 Biberach (DE)
(74) Vertreter: Hammann, Heinz
(86) Internationale Anmeldenummer: PCT/EP2007/056451
(87) Internationale Veröffentlichungsnummer: WO 2008/000780

(56) Entgegenhaltungen:
- WO-A-02/11695
- WO-A-95/31479
- WO-A-96/32096
- WO-A-97/03649
- US-B1- 6 372 258
- US-B1- 6 956 021

## Beschreibung

### HINTERGRUND DER ERFINDUNG

### TECHNISCHES GEBIET

Die Erfindung betrifft phenylalanin-haltige Pulver insbesondere sprühgetrocknete Pulver, die mindestens Phenylalanin und ein Protein enthalten, wobei das Protein bevorzugt ein Wirkstoff und insbesondere ein pharmazeutischer Wirkstoff ist. Die erfinderischen Pulver enthalten einen Phenylalaninanteil von mindestens 30% (w/w), bevorzugt 40% (w/w) und optional mindestens einen zweiten pharmazeutisch verträglichen Hilfsstoff, nämlich einen Zucker, der die Proteinstabilität verbessert.

Die Erfindung betrifft weiterhin ein Verfahren zur Herstellung dieser phenylalanin-haltigen Pulver sowie deren Verwendung insbesondere als inhalative Arzneimittel. Bevorzugte Proteine sind pharmazeutische Wirkstoffe wie Antikörper, Teile von Antikörpern, Fusionsproteine mit Antikörpern oder Teilen von Antikörpern, Hormone, Wachstumsfaktoren, Enzyme, Zytokine, Interferone oder Ähnliches zur lokalen Behandlung der Atemwege oder zur systemischen Behandlung.

### HINTERGRUND

In wässrigen Lösungen formulierte Proteinzubereitungen bzw. Wirkstoffe/Wirkstoffzubereitungen unterliegen teilweise Instabilitäten, welche zu verminderter Wirksamkeit bzw. Bioaktivität und erhöhter Toxizität bzw. zu Unverträglichkeiten führen können. Dies trifft sowohl für klassische Pharmazeutika wie auch für Proteine und insbesondere für peptid- bzw. proteinhaltige Wirkstoffe zu. Die Stabilität von Proteinen bzw. pharmazeutischen Wirkstoffen kann durch Veränderung der Struktur (intern) oder durch Zugabe geeigneter Hilfsstoffe (extern) positiv beeinflusst werden.

Ein übliches Verfahren zur externen Stabilisierung von Proteinen oder pharmazeutischen Wirkstoffen ist die Verwendung geeigneter Hilfsstoffe. Hilfsstoffe können grob in folgende Klassen eingeteilt werden: Zucker und Polyole, Aminosäuren, Amine, Salze, Polymere und Tenside.

Zucker und Polyole werden häufig als unspezifische Stabilisatoren eingesetzt. Ihr stabilisierender Effekt wird bei Proteinen oder biologischen Wirkstoffen vornehmlich der "preferential-exclusion " zugeschrieben (Xie and Timasheff, 1997, Biophysical Chemistry, 64(1-3), 25-43; Xie and Timasheff, 1997, Protein Science, 6(1), 211-221; Timasheff, 1998, Advances in protein chemistry, 51, 355-432). Bei der Auswahl von Zuckern werden bei Proteinen oder biologischen Wirkstoffen zumeist reduzierende Zucker vermieden. Saccharose und Trehalose als nicht reduzierende Zucker werden bevorzugt eingesetzt. Weitere Beispiele für geeignete Hilfsstoffe sind Glucose, Sorbitol, Glycerol (Boctor and Mehta, 1992, Journal of Pharmacy and Pharmacology, 44 (7), 600-3; Timasheff, 1993, Annual review of biophysics and biomolecular structure, 22, 67-97; Chang et al., 1993, Pharmaceutical Research, 10(10), 1478-83) und Mannitol (Hermann et al., 1996, Pharmaceutical Biotechnology, 9 (Formulation, Characterization, and Stability of Protein Drugs) 303-328; Chan et al., 1996, Pharmaceutical Research, 13(5), 756-761). Ferner ist bekannt, dass verschiedenste Polymere stabilisierend auf Proteine oder pharmazeutische Wirkstoffe wie zum Beispiel Antikörper wirken. Das in der Vergangenheit häufig eingesetzte humane Serumalbumin (HSA) verfügt zwar über sehr gute stabilisierende Eigenschaften, ist aber aufgrund seiner potentiellen Kontamination mit "blood-bourne" Erregern mittlerweile ungeeignet. Unter den bisher bekannten Polymeren erweist sich Hydroxypropyl-β-Cyclodextrin (HP-β-CD) als besonders geeignet, da es auch parenteral unbedenklich applizierbar ist. Weitere Beispiele sind höhermolekulare Dextrane (18 bis 82 kD), Polyvinylpyrrolidone (PVP), Heparin, Gelatine Typ A und B sowie Hydroxyethyl-Stärke (HES), Heparin, Dextran-Sulfat, Polyphosphorsäure, Poly-L-Glutaminsäure, Poly-L-Lysin.

Neben Zuckern und Polyolen können auch Aminosäuren stabilisierend eingesetzt werden, alleine oder in Kombination mit anderen Hilfsstoffen. Vorzugsweise werden Aminosäuren bei der Stabilisierung von Proteinen verwendet.

Beispielsweise inhibiert die Zugabe von Histidin, Glycin, Natrium-Aspartat (Na-Asp), Glutamat und Lysinhydrochorid (Lys-HCl) die Aggregation von rhKGF in 10 mM Natriumphosphatpuffer (pH 7,0) zusammen mit 5% Mannitol (Zhang et al., 1995, Biochemistry , 34 (27), 8631-41). Die Kombination von Aminosäuren und Propylenglykol verbessert beispielsweise die strukturelle Stabilität von rhCNTF (Dix et al, 1995, Pharmaceutical Research (Supplement), 12, S97). Lysin und Arginin erhöhen die Thermostabilität von IL-1R (Tm-Erhöhung), wogegen Glycin und Alanin destabilisierend wirken (Remmele et al., 1998, Pharmaceutical Research, 15(2), 200-208).

Darüber hinaus lässt sich die Stabilität von Pulvern mit Proteingehalt oder pharmazeutischen Wirkstoffen durch verschiedene Trocknungsverfahren erhöhen. Die Trocknung erfolgt allerdings zumeist ebenfalls in Gegenwart von Hilfsstoffen, die die Stabilität der Proteine oder Wirkstoffe erhalten und die Eigenschaften der trockenen Pulver verbessern sollen. Ein entscheidender Faktor bei der Stabilisierung durch Trocknung ist die Immobilisierung des Proteins oder Wirkstoffs in einer amorphen Matrix. Der amorphe Zustand besitzt eine hohe Viskosität mit geringer molekularer Beweglichkeit und geringer Reaktivität. Vorteilhafte Hilfsstoffe müssen also in der Lage sein eine amorphe Matrix mit möglichst hoher Glasübergangstemperatur zu bilden, in die das Protein oder der Wirkstoff eingebettet wird. Die Auswahl der Hilfsstoffe hängt somit insbesondere von ihren Stabilisierungsfähigkeiten ab. Darüber hinaus spielen aber auch Faktoren wie die pharmazeutische Unbedenklichkeit des Hilfsstoffs sowie dessen Einfluss auf die Teilchenbildung, die Dispergierbarkeit und die Fließeigenschaft eine entscheidende Rolle, insbesondere wenn es sich um Sprühtrocknungsverfahren handelt.

Die Sprühtrocknung stellt ein besonders geeignetes Verfahren zur Erhöhung der chemischen und physikalischen Stabilität von Proteinen oder peptid-/proteinartigen pharmazeutischen Wirkstoffen dar (Maa et al., 1998, Pharmaceutical Research, 15(5), 768-775). Besonders im Bereich der pulmonalen Therapie wird die Sprühtrocknung vermehrt eingesetzt (US 5,626,874; US 5,972,388; Broadhead et al., 1994, J. Pharm. Pharmacol., 46(6), 458-467), da die inhalative Applikation auch bei der Behandlung von systemischen Erkrankungen mittlerweile eine Alternative darstellt (WO 99/07340). Voraussetzung ist, dass die mittlere aerodynamische Teilchengröße (MMAD = Mass median aerodynamic diameter) der Pulverpartikel im Bereich von 1-10 µm, vorzugsweise 1-7,5 µm liegt, so dass die Partikel in tiefere Lungenabschnitte und somit in den Blutkreislauf gelangen können. Die DE-A-179 22 07 beschreibt beispielhaft die Herstellung von entsprechenden Sprühtrocknungspartikeln. Mittlerweile sind eine Vielzahl an Verfahren zur Herstellung entsprechender Pulver beschrieben (WO 95/31479; WO 96/09814; WO 96/32096; WO 96/32149; WO 97/41833; WO 97/44013; WO 98/16205; WO 98/31346; WO 99/66903; WO 00/10541; WO 01/13893; Maa et al., 1998, *supra*; Vidgrén et al., 1987, Int. J. Pharmaceutics, 35, 139-144; Niven et al., 1994, Pharmaceutical Research, 11(8), 1101-1109).

Als Hilfsstoffe bei der Sprühtrocknung eignen sich ebenfalls Zucker und deren Alkohole (z.B. Trehalose, Lactose, Saccharose oder Mannitol) sowie verschiedene Polymere (Maa et al., 1997, Pharm. Development and Technology, 2(3), 213-223; Maa et al., 1998, supra; Dissertation Adler, 1998, Universität Erlangen; Costantino, et al., 1998, J. Pharm. Sci., 87(11), 1406-1411). Die vorwiegend eingesetzten Hilfsstoffe haben allerdings verschiedene Nachteile. Der Zusatz von Trehalose und Mannitol beispielsweise verschlechtert die Fließeigenschaften von Sprühtrocknungsformulierungen (C. Bosquillon et al., 2001 Journal of Controlled Release, 70(3), 329-339). Sprühgetrocknete Trehalose verursacht oftmals ein starkes Verkleben der resultierenden Partikel (L. Mao et. al, 2004 Respiratory Drug Delivery IX, S. 653-656). Damit verbunden sind verfahrenstechnische Probleme hinsichtlich der Ausbeuten an Pulver und der Robustheit des Verfahrens, sowie eine Verschlechterung der Bioverfügbarkeit der Pulver bei pulmonalen Anwendungen bedingt durch eine Reduzierung der erzielbaren Feinpartikelfraktion. Mannitol neigt zudem bei einem Gehalt von mehr als 20 Gewichtsprozent zur Rekristallisation (Costantino et al., 1998, *supra*), wodurch stabilisierende Effekte dramatisch abnehmen. Lactose, ein häufig verwendeter Hilfsstoff, verbessert zwar die Fließeigenschaften von Sprühtrocknungsformulierungen (C. Bosquillon et al., 2001, *supra*), ist aber insbesondere bei der Formulierung von Proteinen oder peptid-/proteinhaltigen Wirkstoffen problematisch, da Lactose aufgrund ihrer reduzierenden Eigenschaft destabilisierende Maillard-Reaktionen mit Peptiden/Proteinen eingehen kann.

Neben der Proteinstabilisierung durch Hilfsstoffe steht aber auch die Optimierung physikochemischer Eigenschaften sprühgetrockneter Pulver im Fokus der Rezepturentwicklung. Im Besonderen neigen Pulver, insbesondere sprühgetrocknete Pulver, zu kohäsivem und adhäsivem Verhalten. Eine wichtige Ursache liegt zum einen in der für die pulmonale Anwendung notwendigen Partikelgröße von < 10 µm. Bei diesen geringen Teilchengrößen überwiegen partikuläre Wechselwirkungen, wie z.B. Van-der-Waals Kräfte, Kapillarkräfte, Dipolwechselwirkungen und elektrostatische Wechselwirkungen, gegenüber Gravitationskräften. [I. Zimmermann, Pharmazeutische Industrie, Springer-Verlag] Während Kapillarkräfte bedingt durch Wasserdampfkondensation durch eine geeignete Lagerung der Pulver bei reduzierten Feuchte kontrollierbar ist, erweisen sich die Van-der-Waals Kräfte sowie die elektrostatischen Wechselwirkungen zwischen den (sprühgetrockneten) Partikeln als große Herausforderung.

Die interpartikulären Wechselwirkungen können durch eine Hydrophobisierung der Partikeloberfläche reduziert werden. Hierzu können hydrophobe Substanzen als Additiv mit dem Protein oder Wirkstoff und weiteren geeigneten Hilfsstoffen gelöst und sprühgetrocknet werden. Als Stand der Technik wird für die Hydrophobisierung der Oberflächen unter anderem die hydrophobe Aminosäure L-Leucin (L. Mao et. al, 2004 Respiratory Drug Delivery IX, S. 653-656, AR.Najafabadi et al.,2004, Int J Pharm. 2004 Nov 5;285(1-2):97-108.) eingesetzt. Da bei diesem Verfahren nur die Oberflächenbelegung modifiziert werden soll, liegt der dafür notwendige Anteil an L-Leucin bei lediglich 5-10 Gewichtsprozenten (% w/w). Eine Erhöhung des Aminosäureanteils führt häufig zu unerwünschten Kristallisationseffekten, wodurch das Protein geschädigt wird (Dissertation Richard Fuhrherr, 2005 Uni LMU München). Die Zugabe anderer Aminosäuren wie z.B. DL-Asparagin, DL-Arginin, DL-Methionin, DL-Phenylalanin und DL-Tryptophan (N.Y.K. Chew et. al, 2002 Respiratory Drug Delivery VIII, S. 743-745) zum Protein und bevorzugt zur Sprühlösung kann einen positiven Einfluss auf das aerodynamische Verhalten der Partikel haben. Neben dem direkten Zusetzen der hydrophoben Substanz zum Protein und insbesondere in die Sprühlösung können die Pulverpartikel in einem weiteren Prozessschritt mit Additiven beschichtet werden. Hierfür besonders geeignet sind L-Leucin, Phospholipide sowie Mg-Stearate (WO2004093848). Potentielle Verfahren für die Beschichtung sind Freifallmischer, z.B. Taumelmischer (US2005152849), aber auch mechanische Mischverfahren wie z.B. das Strahlmahlen (WO2004093848).

Eine übliche Methode zur Verabreichung von Proteinen und Peptiden ist die parenterale Applikation. Hierbei kann der Wirkstoff zum Beispiel intravenös, intramuskulär sowie subkutan gegeben werden. Stand der Technik ist die Verabreichung des Medikaments über eine Kanüle z.B. in Kombination mit einer Spritze, einem Pen oder als Infusion mit einem Infusionsbeutel. Nachteilig hierbei ist, dass Pulverformulierungen vor der Applikation in Flüssigkeit rekonstituiert werden müssen. Die parenterale Appliaktionsform ist des weiteren aufgrund der häufig vorkommenden Nadelphobie bei Patienten nicht beliebt. Aus diesen Gründen müssen parenterale Anwendungen oft vom Arzt appliziert werden.

Im Gegensatz dazu kann die systemische, inhalative Applikationsform vom Patienten selbst angewendet werden.

Proteine/Peptide können durch passive Diffusion oder durch einen aktiven Transport über die Lunge in den Blutkreislauf gelangen. Beim passiven Transport ist die Resorptionsgeschwindigkeit eine Funktion der Molekülgröße des Wirkstoffes [J.S. Patton, Nature Biotechnology, 16, 141ff, 1998].

Während bei kleinen Proteinen wie zum Beispiel beim Insulin gute Bioverfügbarkeiten festgestellt werden konnten (J.S. Patton, 1999 Advanced Drug Delivery Review, 35, 235-247) zeigen größere Proteine und im besonderen Antikörper in der Regel eine sehr geringe Resorptionsrate. Um dennoch eine effiziente Medikationsform zu entwickeln, müssen größere Proteinen über spezifische Mechanismen aktiv durch das Lungenepithel transportiert werden. Eine Möglichkeit des aktiven Transportes von Antikörpern durch das Lungenepithel ist der neonatale Fc-Rezeptor (A. Bitonti, 2004, Respiratory Drug Delivery IX, 79-85). Es konnte festgestellt werden, dass dieser Rezeptor in der Lunge nicht nur bei Neugeborenen, sondern auch bei Kindern und Erwachsenen in ausreichend großer Zahl zur Verfügung steht und für den aktiven Transport von Wirkstoffen genutzt werden kann.

Bei der Herstellung von Pulvern mit Proteingehalt für medizinische Anwendungen, insbesondere von sprühgetrockneten Pulvern bzw. Proteinzusammensetzungen, besteht eine besondere Herausforderung darin, neben einer guten Proteinstabilität auch ein möglichst vorteilhaftes aerodynamisches Verhalten zu erzielen, damit die Pulver bzw. ihre Partikel, insbesondere sprühgetrocknete Pulver und Partikel, tief in die Lunge und damit leicht in die Blutbahn gelangen können.

In neuerer Zeit werden immer mehr inhalierbare pharmazeutische Zusammensetzungen entwickelt (inhalierbares Insulin als Entwicklungsprodukte der Firmen Aradigm, Mannkind oder Kos, K. Corkery, Respiratory Care, 45, 831ff, 2000) bzw. sind schon auf dem Markt (z.B. Pulmozyme® als inhalative Form der rekombinanten, humanen Deoxyribonuclease I (rhDNase) oder Exubera als inhalative Form von humanem Insulin, siehe US5997848) und das zur Behandlung verschiedener Erkrankungen. Es hat sich gezeigt, dass gewisse Medikamente leicht in der Lunge über die Alveolen direkt in den Blutkreislauf absorbiert werden. Die inhalative Applikation ist besonders vielversprechend für die Verabreichung von Makromolekülen wie Proteinen, Polypeptiden und Nukleinsäuren, die mittels anderer Wege (z.B. oral) schwierig zu verabreichen sind. Eine solche inhalative Applikation kann sowohl für systemische Erkrankungen als auch für lokale Erkrankungen der Lunge wirksam eingesetzt werden.

Pulmonale Medikamentenapplikation kann mittels verschiedener Verfahren erreicht werden, z.B. mittels Flüssigvernebler (liquid nebulizers), Treibgasbasiertem Inhalator (aerosol-based metered dose inhaler =MDI), und Trockenpulver-Dispersionsgerät (dry powder dispersion device). Die Entwicklung Treibgas-basierter Applikationsformen ist mit einer Vielzahl von Problemen verbunden. So dürfen die etablierten Chlorfluorkohlenwasserstoffen (CFC's) wegen ihrer ozonschädigenden Eigenschaften nicht mehr eingesetzt werden. Als Ersatz können alternative Treibgase (HFA-143a / HFA227) verwendet werden. Die alternativen Treibgase zeigen jedoch oftmals eine im Vergleich zu den CFC's reduzierte Löslichkeit des Wirkstoffes. Zusätzlich ist bei Herstellung von Suspensionen die Stabilität der Suspension kritisch, so dass weitere Hilfsstoffe als Vermittler zwischen dem Treibgas und dem Partikel notwendig sind. Hohe Dosiseinstellungen, wie sie oftmals bei Antikörpern notwendig sind, sind mit MDI's nur schwer zu erreichen. Diese genannten Faktoren bedingten, dass MDI's für Peptid- und Proteinrezepturen immer weniger bevorzugt werden. Trockenpulver-Dispersionsgeräte, welche nicht auf Treibgas Aerosol-Technologie angewiesen sind, sind vielversprechend bei der Applikation von Medikamenten, die problemlos als Trockenpulver formuliert werden können.

Viele ansonsten labile Makromoleküle können in Form von Pulver, insbesondere lyophilisierten oder sprühgetrockneten Pulvern, allein oder in Kombination mit geeigneten Hilfsstoffen stabilisiert werden. Die Fähigkeit zur Verabreichung pharmazeutischer Zusammensetzungen als Trockenpulver birgt jedoch einige Probleme. Die Dosierung vieler pharmazeutischer Zusammensetzungen ist oft kritisch. Daher ist es notwendig, dass jedes System zur Verabreichung von Trockenpulver die beabsichtigte Dosis auch wirklich akurat, präzise und zuverlässig verabreicht. Dies ist bei bisherigen Systemen nicht zuverlässig der Fall. Darüber hinaus sind viele pharmazeutische Zusammensetzungen sehr teuer. Daher ist die Fähigkeit zur effizienten Ausbringung des Trockenpulvers wichtig. Es ist ebenfalls wichtig, dass das Pulver vor der Inhalation durch den Patienten leicht dispergierbar (flugtauglich) ist , damit eine adäquate Verteilung und eine systemische Absorption gesichert ist. Auch diese Punkte sind in den meisten herkömmlichen Pulvern mit Proteingehalt oder pharmazeutischem Wirkstoff nicht optimal erfüllt.

Es ergibt sich daher das Problem, dass in bisher verwendeten Pulvern mit Proteinanteil, insbesondere bei sprühgetrockneten Pulvern bzw. Proteinzusammensetzungen mit pharmazeutischem Wirkstoff, eine effiziente und optimale pulmonale Verabreichung nicht möglich ist. Es konnte bei bisher verwendeten Pulvern zwar eine gute Proteinstabilität erreicht werden, aber keine optimalen aerodynamischen Eigenschaften. Beispielsweise bewirken hohe Anteile an Antikörper im Pulver, insbesondere im sprühgetrockneten Pulver, eine starke Agglomeration der Primärpartikel. Diese Agglomerate lassen sich nur schwer dispergieren, wodurch das aerodynamische Verhalten negativ beeinflusst wird (Doktorarbeit Stefanie Schüle, Uni LMU 2005).

Damit muss die Dosierung des zu verabreichenden Proteins oder des pharmazeutischen Wirkstoffs deutlich höher erfolgen, als eigentlich notwendig, denn vom eingesetzten Wirkstoff erreichen nur Bruchteile den eigentlichen Zielort in der Lunge. Damit ist auch die Gefahr der Nebenwirkungen höher als bei einer effizienten Dosierung.

Es ergibt sich somit die Aufgabe, alternative Pulver, insbesondere sprühgetrocknete Pulver bzw. Proteinzusammensetzungen, bereit zu stellen, die neben einer ausreichenden Proteinstabilität auch sehr gute bzw. verbesserte aerodynamische Eigenschaften aufweisen.

Eine weitere Aufgabe der Erfindung besteht darin, entsprechende alternative Pulver, insbesondere sprühgetrocknete Pulver bzw. Proteinzusammensetzungen, für die inhalative Applikation bereit zu stellen und zwar insbesondere für pharmazeutische bzw. medizinische Anwendungen.

Die der Erfindung zugrunde liegenden Aufgaben werden durch die nachfolgenden Ausführungen sowie durch die in den Patentansprüchen dargestellten Gegenstände und Verfahren gelöst.

### ZUSAMMENFASSUNG DER ERFINDUNG

Die vorliegende Erfindung betrifft Pulver, insbesondere sprühgetrocknete Pulver, enthaltend ein Protein und Phenylalanin sowie optional einen Zucker, **dadurch gekennzeichnet, dass** das Pulver mindestens 30 % (w/w) Phenylalanin, bevorzugt mindestens 40 % (w/w) Phenylalanin enthält.

Die vorliegende Erfindung betrifft ferner eine pharmazeutische Zusammensetzung, insbesondere eine sprühgetrocknete Zusammensetzung, enthaltend ein Protein und Phenylalanin sowie optional einen weiteren Hilfsstoff wie einen Zucker oder ein Polyol, **dadurch gekennzeichnet, dass** das Pulver mindestens 30 % (w/w) Phenylalanin, bevorzugt mindestens 40 % (w/w) Phenylalanin enthält.

Die vorliegende Erfindung betrifft weiterhin ein Verfahren zur Herstellung eines Pulvers **dadurch gekennzeichnet , dass**
a) eine Phenylalaninlösung hergestellt wird,
b) mindestens ein Protein und optional mindestens ein weiterer Hilfsstoff wie ein Zucker oder ein Polyol hinzugefügt werden,
c) die so erhaltene Lösung oder Suspension bei einer Einströmtemperatur von vorzugsweise 90 - 200 °C und einer Ausströmtemperatur von vorzugsweise 40 - 150 °C versprüht wird und
d) die entstandenen Partikel vom Trocknungsgas abgetrennt werden.

Die vorliegende Erfindung betrifft zudem Pulver zur Verwendung als Arzneimittel und insbesondere als inhalatives Arzneimittel zur Behaudlung von Atemwegserkrankungen oder systemischen Erkrankungen sowie die Verwendung des beschriebenen Pulvers zur Herstellung eines Medikaments zur Behandlung von Atemwegserkrankungen oder systemischen Erkrankungen wie Lungenkrebs, Lungenentzündung, cystische Fibrose, COPD (chronisch obstruktive Lungenerkrankung), Asthma, anti-inflammatorische Erkrankungen, virale Erkrankungen z.B. durch den respiratorisch-syncytial Virus (RSV).

Es konnte gezeigt werden, dass sich binäre und ternäre Pulver mit Proteinanteil bezüglich ihres aerodynamischen Verhaltens und ihrer Proteinstabilisierung nach Sprühtrocknung sehr gut zur Herstellung alternativer, bevorzugt sprühgetrockneter Pulver bzw. Proteinzusammensetzungen mit hervorragenden aerodynamischen Eigenschaften eignen. Hierbei ist die Hauptkomponente Phenylalanin und die optionale weitere Komponente ein im Vergleich zum Phenylalanin gut wasserlöslicher Hilfsstoff wie ein Zucker oder ein Polyol.

Entscheidend für die Pulverherstellung ist der hohe Anteil an Phenylalanin. Durch seine geringe Löslichkeit und die hohe Hydrophobizität reichert sich das Phenylalanin an der Partikeloberfläche an und ist dementsprechend für die Oberflächenstruktur und Partikelmorphologie verantwortlich. Gut wasserlösliche Komponenten, wie z.B. die Zucker Lactosucrose (LS90P) oder Saccharose sowie das Protein sollten demzufolge hauptsächlich im Inneren des Kerns ausfallen und eine amorphe Matrix bilden.

Es, konnte weiterhin gezeigt werden, dass andere Aminosäuren mit ähnlichen Eigenschaften hinsichtlich Hydrophobizität und Löslichkeit (z.B. Valin, Leucin oder Isoleucin) kein entsprechend gutes aerodynamisches Verhalten der Pulver liefern und dementsprechend ungeeignet für die Herstellung solcher Pulverformulierungen mit mindestens 30 % (w/w) Phenylalanin, bevorzugt mindestens 40 % (w/w) Phenylalanin bzw. den weiterhin genannten Phenylalanin % (w/w) Anteilen sind.

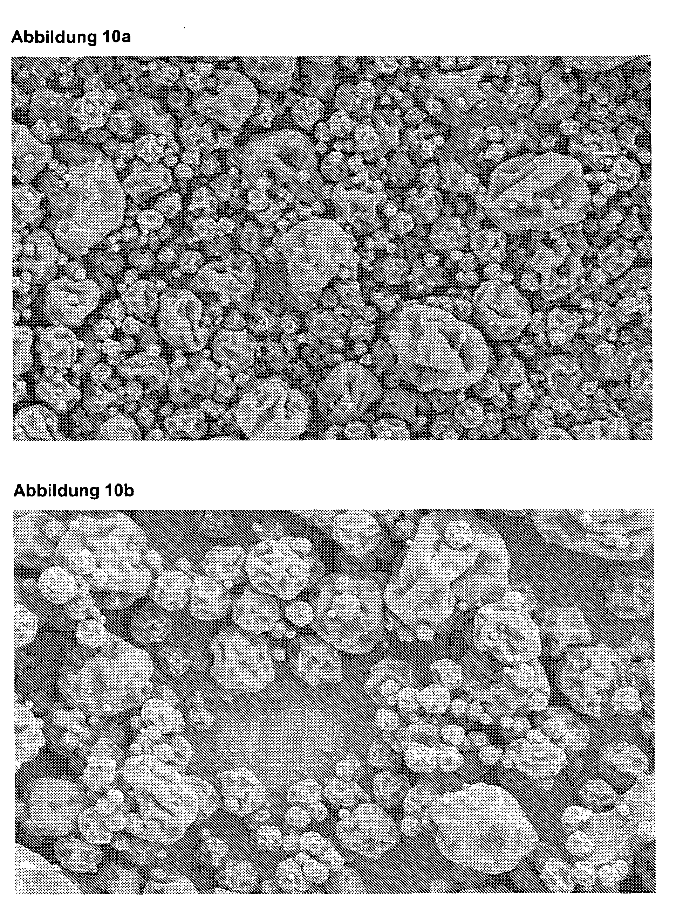

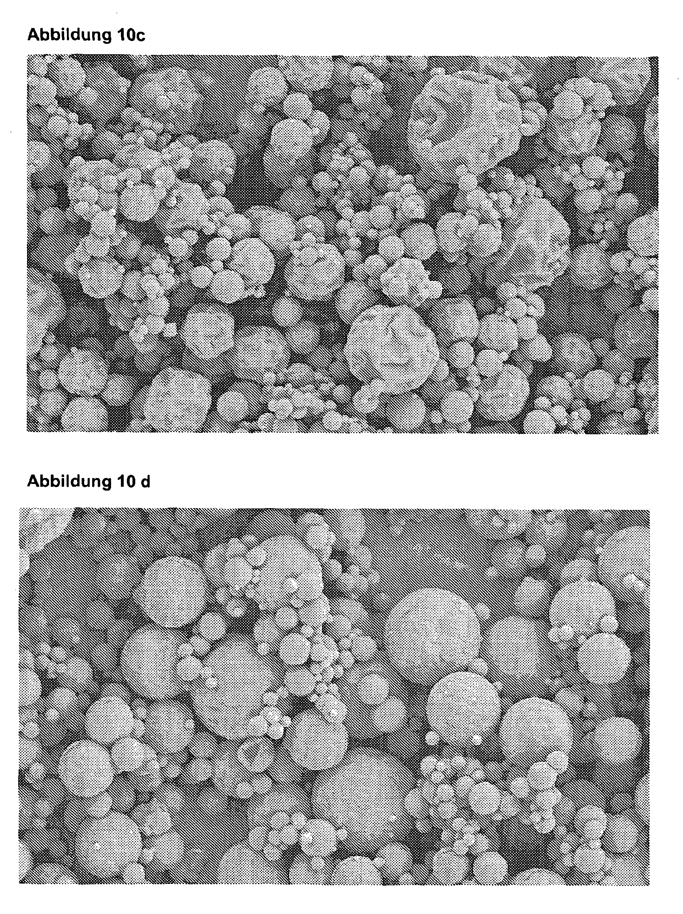

Es konnte weiterhin gezeigt werden, dass die Partikelmorphologie stark vom Phenylalanin-Anteil im sprühgetrockneten Pulver abhängt. Bei 50% (w/w), 40% (w/w) und 30% (w/w) Phenylalanin-Anteil liegen stark gefaltete, rosinen-artige Partikel vor (Abbildung 10a - 10c). Bei einer Reduzierung des Phenylalanin-Anteils auf 20% nimmt die Faltungsintensität stark ab. Die Änderung der Partikelmorphologie korreliert mit der Verschlechterung des aerodynamischen Verhaltens des Pulvers. Das bedeutet, das der positive Effekt des Phenylalanins beim Sprühtrocknen von Sprühlösungen erst ab 30% (w/w) deutlich wird.

Tests mit anderen aromatischen Aminosäuren ergaben Folgendes.

Tyrosin weist eine zu geringe Wasserlöslichkeit auf, um als Formulierungskomponente in Frage zu kommen.

Bei Tryptophan konnte lediglich eine Pulverformulierung mit 20% Tryptophan-Anteil hergestellt werden. Bei diesen geringen Anteilen konnte kein technischer Vorteil des Tryptophans für die Sprühtrocknung und im Besonderen im aerodynamischen Verhalten festgestellt werden.

Histidin-haltiges Pulver ist verglichen zum Phenylalanin-haltigen Pulver sehr empfindlich gegenüber Luftfeuchtigkeit. Daher liegt ein wesentlicher Vorteil des Phenylalanin-haltigen Pulvers gegenüber dem Histidin-haltigen Pulver in der geringeren Feuchteempfindlichkeit. Während die FPF des Histidin-haltigen Pulvers nach einer Exposition mit 50% relativer Luftfeuchte einbricht, kommt es beim Phenylalanin-haltigen Pulver nach Feuchteexposition sogar zu einer Optimierung der FPF. Ein entsprechendes Verhalten ist auch für die ausgebrachte Masse zu erkennen. Beim Histidin-haltigen Pulver nimmt die ausgebrachte Masse bei Feuchteexposition ab, beim Phenylalain-haltigen Pulver dagegen zu.

Zusammenfassend kann festgestellt werden, dass die positiven Eigenschaften des Phenylalanins auf die Sprühtrocknung nicht durch andere aromatische Aminosäuren erzielbar sind.

Weiterhin können Kristallisationsinhibitoren wie HSA die Partikeleigenschaften von Pulvern verbessern. Kristallisationsinhibitoren unterstützen die Bildung einer amorphen Matrix im Inneren des Partikelkerns, wo sich die gut wasserlöslichen Komponenten, wie z.B. Zucker sowie das Protein befinden.

Es konnte weiterhin gezeigt werden, dass durch eine geschickte Wahl der Hilfsstoffe der positive Effekt des Phenylalanins auf den Sprühtrocknungsprozess weiter verbessert werden kann. Hierbei ist der weitere Hilfsstoff nicht auf eine Stoffklasse beschränkt. Es kann sich, wie in diesem Beispiel gezeigt, um einen Zucker oder Zuckeralkohol, eine Aminosäure oder auch um ein Polymer handeln. Entscheidend für die Verwendung des weiteren Hilfsstoffes ist die Stabilisierung des Proteins bei der Sprühtrocknung. Es zeigt sich zudem, dass durch die Zugabe eines weiteren Hilfsstoffes das Protein verglichen zu binären Gemischen aus Phenylalanin und IgG1 stabilisiert werden kann.

Die Erfindung ergibt sich nicht aus dem Stand der Technik.

Zur Verbesserung der Partikeleigenschaften von pharmazeutischen Pulvern zur pulmonalen Applikation insbesondere nach Sprühtrocknung sind Verfahren aus dem Stand der Technik bekannt wie z.B. die Möglichkeit zur Hydrophobisierung der Partikeloberflächen aus US 6,372,258 und US2005/0152849. US 6,372,258 verwendet hydrophobe Aminosäuren, darunter Phenylalanin zur Herstellung von sprühgetrockneten Pulvern.

Bei diesem Verfahren werden in die Sprühlösung neben dem Protein oder Wirkstoff hydrophobe Aminosäuren gegeben und in gelöster Form versprüht und getrocknet. Durch die hydrophoben Eigenschaften der Aminosäure kommt es im zerstäubten Tropfen zu einer Anreicherung der Aminosäure an der Tropfenoberfläche und damit schließlich auch zu einer Anreicherung an der Partikeloberfläche. Durch die Hydrophobisierung reduziert sich die Affinität des Wasser zum Pulver. Damit verbunden ist eine Verringerung der Kapillarkräfte bedingt durch eine niedrigere Wasserdampfkondensation sowie eine Verringerung der Dipol-Wechselwirkungen.

US 6,372,258 beschreibt aber weder die besonders vorteilhafte aerodynamische Wirkung von Phenylalanin in Mindestmengen von 30% (w/w), bzw. 40 % (w/w) im Vergleich mit anderen hydrophoben Aminosäuren wie Leucin oder Tryptophan noch die besonders vorteilhaften Wirkungen von ternären Komplexen aus 30% (w/w), bevorzugt 40 % (w/w) Phenylalanin, einem weiteren Hilfsstoff, bevorzugt einem Zucker oder Polyol, und einem Protein, insbesondere einem Proteinwirkstoff.

Bei WO970364 bzw. US2005/0152849 liegt der Schwerpunkt auf dem Vermischen des Wirkstoffes mit einem sogenannten Anti-adhärenz.

Die Anmeldungen beschreiben unter anderem die Verwendung von Leuzin als anti-adhärentes Material, welches zur Beschichtung der Partikel dient, um die Agglomeration der Partikel zu verhindern. Laut US2005/0152849 sollen jedoch nicht mehr als 10 % des Pulvers aus dem Hilfsstoff bestehen.

In EP 0913177 wird ein Verfahren zur Herstellung trockener, amorpher Produkte enthaltend biologisch aktive Materialien mittels Konvektionstrocknung, insbesondere Sprühtrocknung beschrieben. In den offenbarten Gemischen aus Protein (EPO), Zucker und Aminosäuren (z.T. auch mit Tween 20) ist der Anteil an Zucker jedoch stets höher als der Anteil der Aminosäuren. Zudem werden stets 2 Aminosäuren eingesetzt. Weiterhin wurden entgegen den Versuchen in EP 0913177 in der vorliegenden Erfindung die Aminosäure nicht auf ihren isoelektrischen Punkt titriert. Das besonders vorteilhafte aerodynamische Verhalten (FPF, ausgebrachte Masse) der vorliegenden erfindungsgemäßen Pulver ist nicht auf den isoelektrischen Punkt des Phenylalanins beschränkt. Die bei unterschiedlichen pH-Werten hergestellten Pulver waren jeweils teilkristallin. Demnach ist der pH-Wert der Sprühlösung nicht entscheidend für die Pulvereigenschaften (Dispergierbarkeit / Inhalierbarkeit) und der Sprüheigenschaft des Phenylalanins. Die Proteinstabilisierung hängt zwar vom pH-Wert der Sprühlösung ab (der eingesetzte Antikörper ist bei niedrigen pH-Werten stabiler), dennoch kann auch bei hohen pH-Werten von 9,0 eine Proteinstabilisierung im besonderen im Vergleich zu binären Zusammensetzungen erreicht werden.

Bei WO0033811 werden im Besonderen Aminosäure-haltige Partikel mit einer geringen Dichte (nicht mehr als 0,1 g/cm³) hergestellt. Ein mögliches Verfahren ist die Sprühtrocknung. Jedoch überschreitet hier zum einen der Aminosäure-Anteil nicht die 20 % Marke, zum anderen liegt der Schwerpunkt der Offenbarung bei WO0033811 beim Leucin. Phenylalanin wird in WO003381 nicht erwähnt.

In WO0211695 werden protein-haltige, sprühgetrocknete Inhalationspulver beschrieben, die weniger als 30% eines Zusatzstoffes enthalten. Der in WO0211695 beschriebene Zusatzstoff kann eine beliebige Aminosäure sein. Eine Besonderheit bzw. Bevorzugung bei Einsatz der Aminosäure Phenylalanin beschreibt W00211695 nicht.

In WO9632096 werden Inhalationspulver beschrieben, welche Protein, einen Wirkstoff und 50-99.9% eines Hilfsstoffes enthalten. Auch in WO0211695 kann der beschriebene Zusatzstoff eine beliebige Aminosäure sein. Eine direkte Offenbarung der Aminosäure Phenylalanin enthält WO0211695 nicht.

In JP62281847 sind Sprühtrocknungen mit reinem Phenylalanin durchgeführt worden. Der Fokus lag hierbei jedoch nicht auf der Inhalation. Die hergestellten Partikelgrößen sind demnach wesentlich höher.

Der Stand der Technik kennt weiterhin die Versprühung der Aminosäuren Asparagin, Arginin, Leucin, Methionin, Phenylalanin und Tryptophan mit einem Protein (N.Y. K. Chew et. al, 2002 Respiratory Drug Delivery VIII, S. 743-745). Der Aminosäure-Anteil betrug in der Regel 5 % (w/w). Ausnahme war Leucin, hier wurde zusätzlich noch 10 % (w/w) Aminosäure Anteil versprüht. Je nach Flussrate und Gerät wurden bei allen Aminosäuren eine Verbesserung der FPF festgestellt. Der beste Effekt lag jedoch beim Leucin vor. Bei Verwendung eines Dinkihalers und einer Flußrate von 120 Umin konnte auch beim Phenylalanin FPFs zwischen 55-60 % (w/w) gemessen werden. Ein Abgrenzung zur Diensterfindung liegt erneut im Anteil an Phenylalanin. Des weiteren wurden bei der Arbeit von Chew at al. keine ternären Gemische eingesetzt.

### BESCHREIBUNG DER ABBILDUNGEN

Sämtlich in den Beschreibungen aufgeführten Prozentangaben beziehen sich auf Konzentrationsangaben und Zusammensetzungen der trockenen Feststoffe insbesondere in einem durch Sprühtrocknung erzielten Pulver (w/w).

### ABBILDUNG 1:

REM Aufnahmen von sprühgetrocknetem Pulver enthaltend einen IgG1 Antikörper und eine Aminosäure:

Die Aufnahmen wurden mit einem Rasterelektronenmikroskop (SUPRA 55 VP, Fa. Zeiss SMT, Oberkochen) aufgenommen. Hierzu wurden die Pulverproben direkt auf geeignete Probenteller aufgestäubt. Überschüssiges Material wurde abgeklopft und abgeblasen. Anschließend wurden die Proben zur Sicherstellung einer ausreichenden elektrischen Leitfähigkeit mit 10 nm Gold-Palladium beschichtet.

Die Detektion zur Darstellung der Bilder erfolgte über Sekundärelektronen.
**a)** Zusammensetzung des sprühgetrockneten Pulvers: 90 % Valin/10 %IgG1
   Vergrößerung: 5000 fach
   Abstand Pulver-Kathode: 8mm
   Blendengröße: 20 µm
   Beschleunigungsspannung: 6 kV
   Vakuum: 5,73e-005 Pa
**b)** Zusammensetzung sprühgetrockneten Pulvers: 90 % Isoleucin/10%IgG1
   Vergrößerung: 3000 fach
   Abstand Pulver-Kathode: 8 mm
   Beschleunigungsspannung: 6 kV
   Vakuum: 5,47e-005 Pa
**c)** Zusammensetzung sprühgetrockneten Pulvers: 90 % Phenylalanin/10 %IgG1
   Vergrößerung: 5000 fach
   Abstand Pulver-Kathode: 8 mm
   Beschleunigungsspannung: 6 kV
   Vakuum: 5,73e-005 Pa

### ABBILDUNG 2:

Vergleich der Hydrophobizität verschiedener Aminosäuren und der Proteinmonomergehalte nach Sprühtrocknung binärer Gemische in Abhängigkeit der Feststoffkonzentration in der Sprühlösung (50 % und 90 % erreichte Löslichkeitsgrenze der Aminosäure):

In dieser Abbildung wird die Proteinstabilisierung nach Sprühtrocknung mit den hydrophoben Anteilen der eingesetzten Aminosäuren verglichen. Für die Angabe der Hydrophobizität von Aminosäuren gibt es mehrere Ansätze (P. Andrew Karplus, Hydrophobicity reganied, Protein science (1997), 6: 1302-1307). Ein gängiger Weg ist die Angabe der freien Enthalpie beim Transfer einer Substanz von einem Solvent ins Wasser (z.B. ΔG°_{trans Okt/Wasser}). Der Nachteil dieser Methode ist die starke Abhängigkeit der Ergebnisse von den Messbedingungen (z.B. Wahl des Solvents). Besonders bei polaren Substanzen können so große Unterschiede in den Ergebnissen vorkommen. Die reine Betrachtung der hydrophoben Flächen ist dagegen unabhängig von den Messbedingungen. Daher sind in dieser Abbildung nur die hydrophoben Anteile bzw. Flächen der Aminosäurereste berücksichtigt. Hierbei werden aliphatischen CH₂-Gruppen eine Enthalpie von 25 cal/Å² und aromatischen CH-Gruppen eine Enthalpie von 16 cal/Å² zugewiesen. Diese Betrachtung berücksichtigt keine polaren Anteile sowie keine durch die Elektronegativität erzeugten induktiven Effekte.

Die Neigung zum Ausbilden von Proteinaggregaten wurde mit einer Ausschlusschromatographie (HP-SEC) ermittelt. Der Ausschluss erfolgte über die Molekülgröße des Proteins bzw. dessen Aggregaten (z.B. Dimere). Es ist bekannt, dass eine Aggregatbildung einhergeht mit einer Proteindestabilisierung.

Zusammensetzungen der sprühgetrockneten Pulver:
- Charge 1:: 10 % IgG1 / 90 % Isoleucin, Feststoffanteil: 3,5 %
- Charge 2: 10 % IgG1 / 90 % Glycin , Feststoffanteil: 20,2 %
- Charge 3: 10 % IgG1 / 90 % Valin , Feststoffanteil: 5,8 %
- Charge 4: 10 % IgG1 / 90 % Phenylalanin, Feststoffanteil: 3,2 %
- Charge 5: 10 % IgG1 / 90 % Asparagin, Feststoffanteil: 2,4 %
- Charge 6: 10 % IgG1 / 90 % Glycin , Feststoffanteil: 11,18 %
- Charge 7: 10 % IgG1 / 90 % Isoleucin, Feststoffanteil: 1,95 %
- Charge 8: 10 % IgG1 / 90 % Valin, Feststoffanteil: 3,21%
- Charge9: 10 % IgG1 / 90 % Phenylalanin, Feststoffanteil: 1,79 %
- Charge 10: 10 % IgG1 / 90 % Asparagin, Feststoffanteil: 1,3 %

Balken: Hydrophobizität der Aminosäure
Raute: Monomergehalt des IgG1-Anitkörpers

### ABBILDUNG 3

### REM-Aufnahmen verschiedener ternärer Pulvergemische enthaltend Phenylalanin, Lactosucrose und einen IgG1-Antikörper

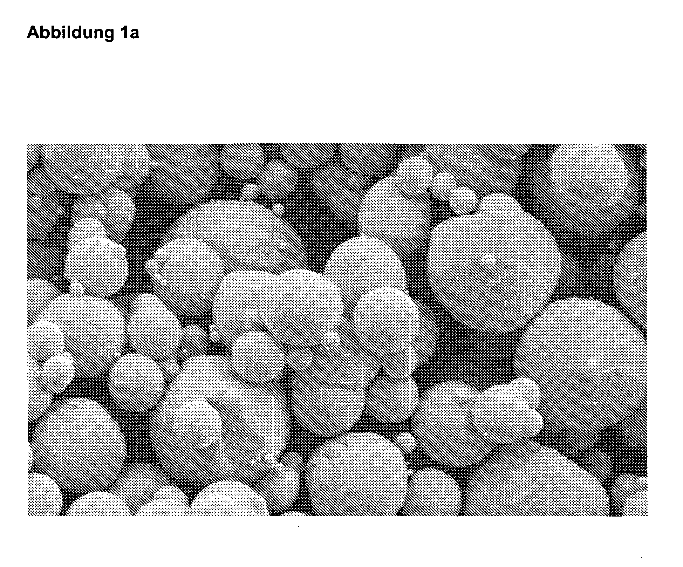

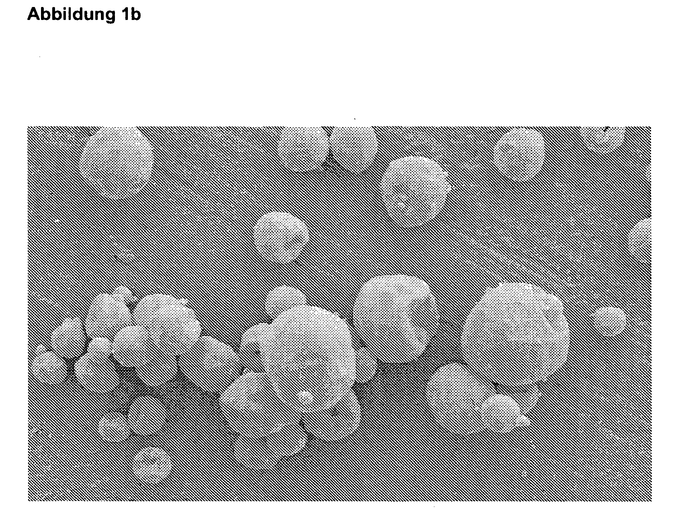

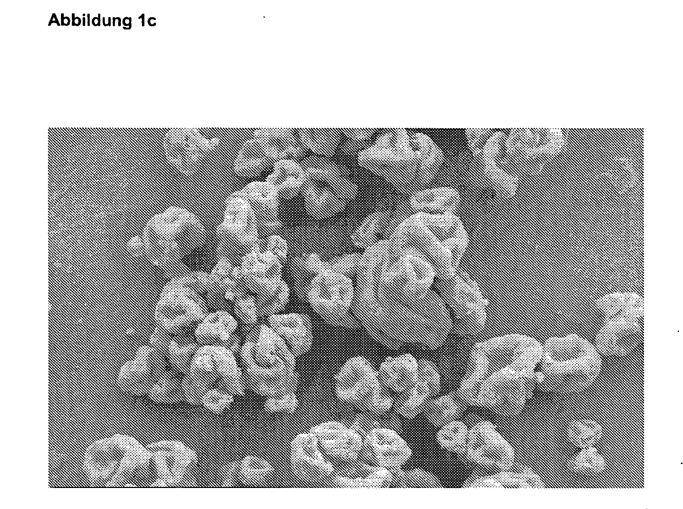

Die Aufnahmen erfolgten wie unter Abbildung 1 beschrieben.
a) Zusammensetzung des sprühgetrockneten Pulvers:
   80 % Phenylalanin / 10% LS90P / 10% IgG1
   Vergrößerung: 5000 fach
   Abstand Pulver-Kathode: 9 mm
   Blendengröße: 10 µm
   Beschleunigungsspannung: 3 kV
   Vakuum: 1,72e-005 Pa
b) Zusammensetzung des sprühgetrockneten Pulvers:
   80 % Phenylalanin / 15% LS90P / 5% IgG1
   Vergrößerung: 5000 fach
   Abstand Pulver-Kathode: 7 mm
   Blendengröße: 10 µm
   Beschleunigungsspannung: 4 kV
   Vakuum: 9,18e-005 Pa
c) Zusammensetzung des sprühgetrockneten Pulvers:
   60 % Phenylalanin / 30 % LS90P / 10 % IgG1
   Vergrößerung: 5000 fach
   Abstand Pulver-Kathode: 8 mm
   Blendengröße: 10 µm
   Beschleunigungsspannung: 4 kV
   Vakuum: 9,18e-005 Pa
d) Zusammensetzung des sprühgetrockneten Pulvers:
   70 % Phenylalanin / 25 % LS90P / 5 % IgG1
   Vergrößerung: 5000 fach
   Abstand Pulver-Kathode: 8 mm
   Blendengröße: 9 µm
   Beschleunigungsspannung: 4 kV
   Vakuum: 9,3e-005 Pa

### ABBILDUNG 4

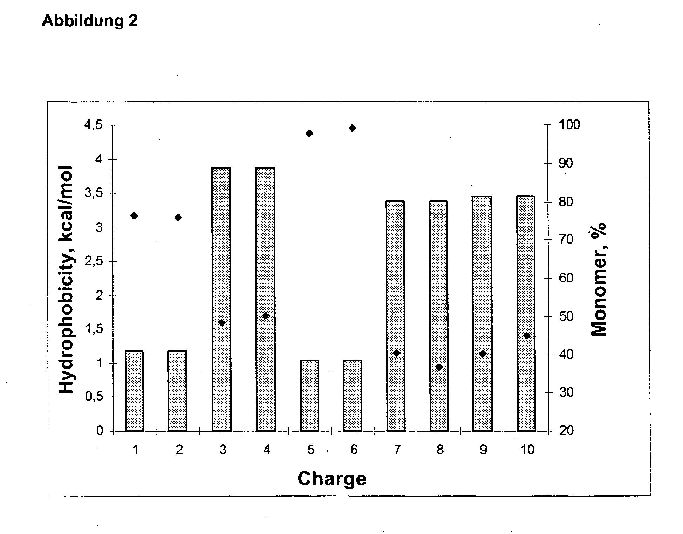

Relativer Monomergehalt bezogen auf den Anfangswert. Der Monomergehalt wurde wie in Abbildung 2 beschrieben bestimmt. Der relative Monomergehalt bezieht sich auf den Anfangswert, der entsprechend 100% gesetzt wird. Diese Darstellung verdeutlicht die Änderung des Monomergehaltes zum Anfangswert und spiegelt somit die Änderung über die Lagerzeit wider.

Raute: sprühgetrocknetes Pulver: 60% Phenylalanin / 10% LS90P / 30% IgG1 Quadrat: sprühgetrocknetes Pulver: 80% Phenylalanin / 10% LS90P / 10% IgG1 Dreieck: sprühgetrocknetes Pulver: 60% Phenylalanin / 30% LS90P / 10% IgG1

### ABBILDUNG 5

Vergleich der Feinpartikelfraktionen verschiedener Pulverzusammensetzungen. Die Feinpartikelfraktion wurde mit einem Einstufenimpaktor (Impactor Inlet, TSI) in Kombination mit dem Aerodynamic Particle Sizer (APS, TSI) bestimmt. Die Trenngrenze der Impaktordüse lag bei 5,0 µm. Zusätzlich zur Feinpartikelfraktion wurde mit dem APS die aerodynamische Teilchengröße sowie die Teilchengrößenverteilung über eine Flugzeitbestimmung ermittelt. Hierzu wurde nach dem Passieren des Sample Induktion Ports das Pulver gesplittet. Ein Anteil von 0,2 % wurde unter isokinetischen Bedingungen in eine kleine Kapillare eingesogen und der Flugzeit Messeinheit zugeführt. Der restliche Anteil wurde für die Bestimmung der Feinpartikelfraktion herangezogen.

Für die Messung wurde das Pulver in Kapseln der Größe 3 abgefüllt und mit einem Inhalator (HandiHaler®, Boehringer Ingelheim) ausgebracht. Die Flussrate zur Ausbringung des Pulvers wurde so eingestellt, dass über den HandiHaler ein Druckabfall von 4 kPa vorherrschte. Das Luftvolumen betrugt entsprechend der PharmEur 4 Liter. Um ein "Rebouncing" der an der Impaktorstufe abgeschiedenen Partikel zu vermeiden, ist bei den Messungen die Impaktorplatte mit einer hochviskosen Brij-Lösung beschichtet worden.
Dunkler Balken: sprühgetrocknetes Pulver: 65 % Dextran1 / 5 % Isoleucin / 30 % IgG1
Heller Balken: sprühgetrocknetes Pulver: 60 % Phenylalanin / 10 % LS90P / 30 % IgG1

### ABBILDUNG 6

Vergleich der relativen Feinpartikelfraktionen verschiedener Pulverzusammensetzungen.

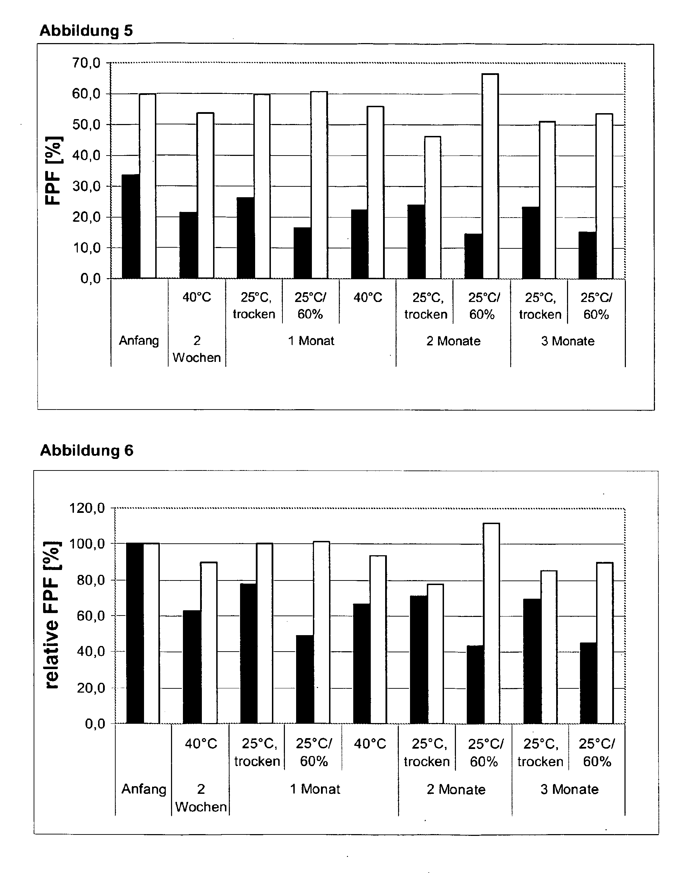

Die relative Feinpartikelfraktion bezieht sich auf die Feinpartikelfraktion des Anfangswertes und spiegelt somit die Änderung der FPF über die Lagerung wieder. Die Feinpartikelfraktion wird entsprechend wie in der Beschreibung zur Abbildung 5 bestimmt.
Dunkler Balken: sprühgetrocknetes Pulver: 65% Dextran1 / 5% Isoleucin / 30% IgG1
Heller Balken: sprühgetrocknetes Pulver: 60% Phenylalanin / 10% LS90P / 30% IgG1

### ABBILDUNG 7

REM-Aufnahmen sprühgetrocknter Pulver enthaltend Phenylalanin bzw. Isoleucin: Die Aufnahmen erfolgten wie unter Abbildung 1 beschrieben.
a) Zusammensetzung des sprühgetrockneten Pulvers:
   60 % Phenylalanin / 10 % LS90P / 10 % IgG1
   Vergrößerung: 250 fach
   Abstand Pulver-Kathode: 7 mm
   Blendengröße: 10 µm
   Beschleunigungsspannung: 6 kV
   Vakuum: 5,35e-005 Pa
b) Zusammensetzung des sprühgetrockneten Pulvers:
   60 % Phenylalanin / 10 % LS90P / 10 % IgG1
   Vergrößerung: 5000 fach
   Abstand Pulver-Kathode: 7 mm
   Blendengröße: 10 µm
   Beschleunigungsspannung: 6 kV
   Vakuum: 5,60e-005 Pa

### ABBILDUNG 8

REM-Aufnahmen sprühgetrocknter Pulver der Zusammensetzung 65 % Dextran 1, 5 % Isoleucin und 30 % IgG1:
Die Aufnahmen erfolgten wie unter Abbildung 1 beschrieben.

a) Zusammensetzung des sprühgetrockneten Pulvers:
   65 % Dextran 1 / 5 % Isoleucin / 30 % IgG1
   Vergrößerung: 250 fach
   Abstand Pulver-Kathode: 9 mm
   Blendengröße: 10 µm
   Beschleunigungsspannung: 4 kV
   Vakuum: 6,70e-005 Pa
b) Zusammensetzung des sprühgetrockneten Pulvers:
   65 % Dextran 1 / 5 % Isoleucin / 30 % IgG1
   Vergrößerung: 7500 fach
   Abstand Pulver-Kathode: 5 mm
   Blendengröße: 10 µm
   Beschleunigungsspannung: 5 kV
   Vakuum: 7,17e-005 Pa

### ABBILDUNG 9

Bestimmung der Feinpartikelfraktion (FPF) und der ausgebrachten Masse sprühgetrockneter Pulver enthaltend verschiedene Phenylalanin-Anteil.

Die Feinpartikelfraktion wurde mit einem Einstufenimpaktor (Impactor Inlet, TSI) in Kombination mit dem Aerodynamic Particle Sizer (APS, TSI) bestimmt (siehe hierzu auch die Beschreibung der Abbildung 5). Die ausgebrachte Masse bezieht sich auf die Masse der eingesetzten Kapsel vor und nach Ausbringung durch den Impactor Inlet / APS. Die Differenz der Masse der Kapsel entspricht der ausgebrachten Masse. Die Methode der Ausbringung ist in Beispiel 5 beschrieben.
Balken: Feinpartikelfraktion (FPF) in Prozent bezogen auf die Einwaage in der Kapsel
Raute: ausgebrachte Masse an Pulver bei Ausbringung in den Inpaktor Inlet / TSI
Pulver 1: Pulver hergestellt durch Sprühtrocknung aus einer Sprühlösung folgender Zusammensetzung: ,29g/100mL Phenylalanin, 1,15g/100mL IgG1, 383mg/100mL LS90P, Puffer: 1,6mM Glycin, 25mM Histidin, pH 4,2
Pulver 2: Pulver hergestellt durch Sprühtrocknung aus einer Sprühlösung folgender Zusammensetzung: ,29g/100mL Phenylalanin, 1,15g/100mL IgG1, 383mg/100mL LS90P, Puffer: 25mM TRIS, pH 7,4
Pulver 3: Pulver hergestellt durch Sprühtrocknung aus einer Sprühlösung folgender Zusammensetzung: ,29g/100mL Phenylalanin, 1,15g/100mL IgG1, 383mg/100mL LS90P, Puffer: 25mM TRIS, pH 9,0

### ABBILDUNG 10

### REM-Aufnahmen sprühgetrocknter Pulver

Die Aufnahmen erfolgten wie unter Abbildung 1 beschrieben.
a) Zusammensetzung des sprühgetrockneten Pulvers:
   50 % Phenylalanin / 20 % LS90P / 30 % IgG1
   Vergrößerung: 2000 fach
   Abstand Pulver-Kathode: 10 mm
   Blendengröße: 10 µm
   Beschleunigungsspannung: 5 kV
   Vakuum: 2,23e-004 Pa
b) Zusammensetzung des sprühgetrockneten Pulvers:
   40 % Phenylalanin / 30 % LS90P / 30 % IgG1
   Vergrößerung: 3000 fach
   Abstand Pulver-Kathode: 10 mm
   Blendengröße: 10 µm
   Beschleunigungsspannung: 5 kV
   Vakuum: 2,23e-004 Pa
c) Zusammensetzung des sprühgetrockneten Pulvers:
   30 % Phenylalanin / 40 % LS90P / 30 % IgG1
   Vergrößerung: 3000 fach
   Abstand Pulver-Kathode: 10 mm
   Blendengröße: 10 µm
   Beschleunigungsspannung: 5 kV
   Vakuum: 2,23e-004 Pa
d) Zusammensetzung des sprühgetrockneten Pulvers:
   20 % Phenylalanin / 50 % LS90P / 30 % IgG1
   Vergrößerung: 3000 fach
   Abstand Pulver-Kathode: 8 mm
   Blendengröße: 10 µm
   Beschleunigungsspannung: 5 kV
   Vakuum: 2,26e-004 Pa

### ABBILDUNG 11

### Bestimmung der Feinpartikelfraktion (FPF) und der ausgebrachten Masse sprühgetrockneter Pulver

Die Feinpartikelfraktion wurde mit einem Einstufenimpaktor (Impactor Inlet, TSI) in Kombination mit dem Aerodynamic Particle Sizer (APS, TSI) bestimmt (siehe hierzu auch die Beschreibung der Abbildung 5). Die ausgebrachte Masse bezieht sich auf die Masse der eingesetzten Kapsel vor und nach Ausbringung durch den Impactor Inlet / APS. Die Differenz der Masse der Kapsel entspricht der ausgebrachten Masse. Die Methode der Ausbringung ist in Beispiel 5 beschrieben.
Balken: Feinpartikelfraktion (FPF) in Prozent bezogen auf die Einwaage in der Kapsel
Raute: ausgebrachte Masse an Pulver bei Ausbringung in den Inpaktor Inlet / TSI
Pulver 1: sprühgetrocknetes Pulver: 60% Phenylalanin, 10% IgG1, 30% LS90P
Pulver 2: sprühgetrocknetes Pulver: 60% Phenylalanin, 10% Lysozym, 30% LS90P
Pulver 3: sprühgetrocknetes Pulver: 60% Phenylalanin, 10% Calcitonin, 30% LS90P

### ABBILDUNG 12

### Bestimmung der Feinpartikelfraktion (FPF) und der ausgebrachten Masse sprühgetrockneter Pulver

Die Feinpartikelfraktion wurde mit einem Einstufenimpaktor (Impactor Inlet, TSI) in Kombination mit dem Aerodynamic Particle Sizer (APS, TSI) bestimmt (siehe hierzu auch die Beschreibung der Abbildung 5). Die ausgebrachte Masse bezieht sich auf die Masse der eingesetzten Kapsel vor und nach Ausbringung durch den Impactor Inlet / APS. Die Differenz der Masse der Kapsel entspricht der ausgebrachten Masse. Die Methode der Ausbringung ist in Beispiel 5 beschrieben.
Balken: Feinpartikelfraktion (FPF) in Prozent bezogen auf die Einwaage in der Kapsel
Raute: ausgebrachte Masse an Pulver bei Ausbringung in den Inpaktor Inlet / TSI
Pulver 1: sprühgetrocknetes Pulver: 60% Phenylalanin, 10% IgG1, 30% Saccharose
Pulver 2: sprühgetrocknetes Pulver: 60% Phenylalanin, 10% IgG1, 30% Mannitol
Pulver 3: sprühgetrocknetes Pulver: 60% Phenylalanin, 10% IgG1, 30% Glycin
Pulver 4: sprühgetrocknetes Pulver: 60% Phenylalanin, 10% IgG1, 30% PVP

### ABBILDUNG 13

### DSC-Messungen zur Bestimmung der Kristallisationsenthalpie des LS90P

Die Kristallisattionsenthalpie wurde durch ein Erfassen der Wärmeströme beim Erwärmen der Pulver ermittelt. Beim Erwärmen eines amorphen Pulvers haben die Bestandteile des Partikels nach dem Durchschreiten der Glasübergangstemperatur eine erhöhte Mobilität und können Kristallisieren. Das Durchschreiten der Glasübergangstemperatur ist ein endothermer Vorgang. Das anschließende Kristallisieren ist dagegen exotherm. Bei weiterer Erhitzung des Pulvers kann es zum Schmelzen oder Zersetzen des Pulvers kommen.

Für die DSC-Messungen wurden wenige Milligramm Pulver in einem Tiegel leicht komprimiert, so dass möglichst ein homogenes und dichtes Pulverbett entsteht. Anschließend wird der Tiegel durch ein Kaltverschweißen verschlossen.

Die Messungen wurden mit einem nicht gelochten Tiegel durchgeführt. Die weiteren Parameter waren:
- Messgerät:: DSC 821 / Mettler Toledo
- Auswertesoftware:: STAR version 4.20
- Ofengas:: Stickstoff / 40mUmin
- Spühlgas:: Stickstoff / 150mL/min
- Tiegel :: Alutiegel, 40µL
- Scanrate:: Temperatur10°C/min

Pulver 1: sprühgetrocknetes Pulver: 60% Phenylalanin / 40% LS90P
Pulver 2: sprühgetrocknetes Pulver : 60% Phenylalanin / 30% LS90P / 10% IgG1
Pulver 3: sprühgetrocknetes Pulver : 60% Phenylalanin / 30% LS90P / 10% Lysozym
Pulver 4: sprühgetrocknetes Pulver : 60% Phenylalanin / 30% LS90P / 10% Calcitonin
Pulver 5: gefriergetrocknetes Pulver: 100% LS90P

### ABBILDUNG 14

### Bestimmung der Feinpartikelfraktion (FPF) sprühgetrockneter Pulver

Die Feinpartikelfraktion wurde mit einem Einstufenimpaktor (Impactor Inlet, TSI) in Kombination mit dem Aerodynamic Particle Sizer (APS, TSI) bestimmt (siehe hierzu auch die Beschreibung der Abbildung 5).

Die ausgebrachte Masse ergibt sich aus der Differenzwiegung der Kapsel vor und nach der Ausbringung durch den Inhalator (HandiHaler®, Boehringer Ingelheim).
Leerer Balken: Bestimmung der FPF direkt nach Sprühtrocknung
Gepunkteter Balken: Bestimmung der FPF nach Feuchteexposition (50%rF bei Raumtemperatur über 20 Stunden)
Dreiecke: ausgebrachte Masse direkt nach Sprühtrocknung
Vierecke: ausgebrachte Masse nach Feuchteexposition (50%rF bei Raumtemperatur über 20 Stunden)
Pulver 1: sprühgetrocknetes Pulver: 20% Tryptophan / 50% LS90P / 30% IgG1
Pulver 2: sprühgetrocknetes Pulver : 20% Histidin / 50% LS90P / 30% IgG1
Pulver 3: sprühgetrocknetes Pulver : 20% Phenylalanin / 50% LS90P / 30% igG1

### DETAILLIERTE BESCHREIBUNG DER ERFINDUNG

### DEFINITIONEN

Im Rahmen dieser Erfindungsbeschreibung verwendete Begriffe und Bezeichnungen haben folgende im Anschluss definierte Bedeutungen. Die Gewichts- und Gewichtsprozentangaben beziehen sich, sofern nichts anderes erwähnt wird, jeweils auf die Trockenmasse der Zusammensetzungen oder den Feststoffgehalt der Lösungen / Suspensionen. Die allgemeinen Ausführungsformen "enthaltend" oder "enthält" schließt die speziellere Ausführungsform "bestehend aus" mit ein. Ferner werden "Einzahl" und "Mehrzahl" nicht begrenzend verwendet.

"Pulver" bezeichnet einen sehr feinen, zerkleinerten Stoff. "Sprühgetrocknete Pulver" meint ein Pulver, welches mittels Sprühtrocknung hergestellt wurde.

"Partikel" bezeichnet ein Teilchen von einem Stoff. In der vorliegenden Erfindung sind mit Partikeln die Partikel in den erfindungsgemäßen Pulvern gemeint. Die Begriffe Partikel und Pulver werden in der vorliegenden Erfindung zeitweise austauschbar verwendet. Mit einem Pulver sind auch dessen Bestandteile, die Partikel, umfasst. Partikel weisen auch auf die Gesamtmenge von Partikeln also das Pulver hin.

Der Begriff "Gemisch" oder "Gemische" im Sinne dieser Erfindung meint sowohl solche Gemische, die sich aus einer echten Lösung aller Komponenten generieren oder aus einer Lösung in der eine oder mehrere der Komponenten suspendiert wurde(n). Der Begriff "Gemische" im Sinne dieser Erfindung meint aber auch solche Gemische, die durch einen physikalischen Mischprozess aus festen Partikeln dieser Komponenten entstanden sind oder die durch Aufbringen einer Lösung oder Suspension dieser Komponenten auf eine oder mehrere feste Komponenten entstanden sind.

Der Ausdruck "Zusammensetzung", meint flüssige, halbfeste oder feste Gemische aus zumindest zwei Ausgangsstoffen.

Der Begriff "pharmazeutische Zusammensetzung" meint eine Zusammensetzung zur Applikation im Patienten.

Der Begriff "pharmazeutisch akzeptable Hilfsstoffe" bezieht sich auf Hilfsstoffe, die optional im Rahmen der Erfindung in der Formulierung enthalten sein können. Die Hilfsstoffe können beispielsweise pulmonal appliziert werden ohne hierbei signifikant ungünstige toxikologische Effekte auf den Probanden oder die Probandenlunge zu haben.

Der Ausdruck "pharmazeutisch akzeptable Salze" umfasst beispielsweise folgende Salze, ist aber nicht begrenzt auf diese: Salze aus anorganischen Säuren, wie Chlorid, Sulfat, Phosphat, Diphosphat, Bromid und Nitratsalze. Des weiteren Salze aus organischen Säuren, wie Malat, Maleat, Fumarat, Tartrat, Succinat, Ethylsuccinat, Citrat, Acetat, Lactat, Methansulfonat, Benzoat, Ascorbat, Para-Toluolsulfonat, Palmoat, Salicylat und Stearat, ebenso wie Estolat, Gluceptat und Lactobionat Salze.

Mit dem Begriff "Wirkstoffe" sind Substanzen gemeint, die in einem Organismus eine Wirkung bzw. eine Reaktion hervorrufen. Wird ein Wirkstoff zu therapeutischen Zwecken am Menschen oder am tierischen Körper angewandt, so bezeichnet man ihn als Arzneimittel oder Medikament.

Unter einem "Proteinwirkstoff" bzw. "Protein-Wirkstoff" wird in der vorliegenden Erfindung ein Wirkstoff verstanden, der strukturell als Protein vorliegt bzw. strukturell ein Protein, Polypeptid oder Peptid darstellt.

Beispiele für Wirkstoffe sind Insulin, Insulin-ähnlicher Wachstumsfaktor, humanes Wachstumshormon (hGH) und andere Wachstumsfaktoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, Interferon (IFN)-alpha, beta, gamma, omega oder tau, Tumornekrosefaktor (TNF) wie z.B. TNF-alpha, beta oder gamma, TRAIL, G-CSF, GM-CSF, M-CSF, MCP-1 und VEGF. Weitere Beispiele sind monoklonale, polyklonale, multispezifische und einzelkettige (*single chain*) Antikörper und Fragmente davon, wie z.B. Fab, Fab', F(ab')₂, Fc und Fc'-Fragmente, leichte (L) und schwere (H) Immunglobulinketten und deren konstante, variable oder hypervariable Regionen sowie Fv- und Fd-Fragmente (Chamov et al., 1999). Die Antikörper können humanen oder nicht-humanen Ursprungs sein. Auch humanisierte und chimäre Antikörper kommen in Frage. Ebenso betrifft dies konjugierte Proteine und Antikörper, welche beispielsweise mit einer radioaktiven Substanz oder einem chemisch-definierten Arzneistoff verbunden sind.

Fab-Fragmente (Fragment antigen-binding = Fab) bestehen aus den variablen Regionen beider Ketten, die durch die angrenzenden konstanten Regionen zusammengehalten werden. Sie können z.B. durch Behandlung mit einer Protease, wie beispielsweise Papain, aus konventionellen Antikörpern erzeugt werden oder aber auch durch DNA-Klonierung. Weitere Antikörperfragmente sind F(ab')₂-Fragmente, die durch proteolytischen Verdau mit Pepsin hergestellt werden können.

Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Da bei diesen Fv-Fragmenten die kovalente Verbindung über die Cysteinreste der konstanten Ketten nicht möglich ist, werden diese Fv-Fragmente oft anderweitig stabilisiert. Dazu werden die variablen Region der schweren und leichten Kette häufig mittels eines kurzen Peptidfragments von ca. 10 - 30 Aminosäuren, besonders bevorzugt 15 Aminosäuren, miteinander verknüpft. Auf diese Weise entsteht eine einzelne Polypeptidkette, in der VH und VL durch einen Peptidlinker miteinander verbunden sind. Solche Antikörperfragmente werden auch als *single-chain* Fv-Fragment (scFv) bezeichnet. Beispiele von scFv-Antikörpern sind bekannt und beschrieben, siehe z.B. Huston et al. (1988).

In den vergangenen Jahren wurden verschiedene Strategien entwickelt um multimere scFv-Derivate herzustellen. Die Intention besteht in der Erzeugung von rekombinanten Antikörpern mit verbesserten pharmakokinetischen Eigenschaften und verstärkter Bindungsavidität. Zur Erreichung der Multimerisierung der scFv-Fragmente werden diese als Fusionsproteine mit Multimerisierungsdomänen hergestellt. Als Multimerisierungsdomänen können dabei z.B. die CH3-Region eines IgGs oder Helixstrukturen ("*coiled coil structure*") wie die Leucin-Zipper-Domänen fungieren. In anderen Strategien wird die Interaktion zwischen den VH-und VL-Regionen des scFv-Fragments für eine Multimerisierung genutzt (z.B. Dia-, Tri- und Pentabodies).

Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VH/VL-Ketten. Die Diabodies können zusätzlich durch eingeführte Disulfidbrücken stabilisiert werden. Beispiele von Diabodies finden sich in der Literatur, z.B. bei Perisic et al. (1994).

Als "Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgG1, als Dimerisierungsregion enthält. Diese verbindet die scFv-Fragmente über eine Hinge-Region, ebenfalls von IgG, und eine Linker-Region. Beispiele solcher Minibodies sind bei Hu et al. (1996) beschrieben.

Mit "Triabody" bezeichnet der Fachmann ein trivalentes homotrimeres scFv-Derivat (Kortt et al., 1997). Die direkte Fusion von VH-VL ohne Verwendung einer Linkersequenz führt zur Ausbildung von Trimeren.

Bei den vom Fachmann als Mini-Antikörper bezeichneten Fragmenten, die eine bi-, tri- oder tetravalente Struktur haben, handelt es sich ebenfalls um Derivate von scFv-Fragmenten. Die Multimerisierung wird dabei über di-, tri- oder tetramere "coiled coil"-Strukturen erzielt (Pack et al., 1993 und 1995; Lovejoy et al., 1993).

Mit dem Begriff "Hilfsstoffe" sind Stoffe gemeint, die einer Formulierung, in der vorliegenden Erfindung einem Pulver, insbesondere einem sprühgetrockneten Pulver, zugefügt werden. Hilfsstoffe haben üblicherweise selbst keine Wirkung, insbesondere keine pharmazeutische Wirkung, und dienen dazu, die Formulierung des eigentlichen Inhaltsstoffs, z.B. eines Wirkstoffes, zu verbessern oder bezüglich eines bestimmten Aspekts (z.B. Lagerstabilität) zu optimieren.

Ein pharmazeutischer "Hilfsstoff meint einen Teil eines Arzneimittels oder einer pharmazeutischen Zusammensetzung, und sorgt unter anderem dafür, dass der Wirkstoff an den Wirkort gelangt und dort freigesetzt wird. Hilfsstoffe haben drei Grundaufgaben: Trägerfunktion, Steuerung der Wirkstoff-Freigabe und Stabilitätserhöhung. Hilfsstoffe dienen auch der Herstellung von Arzneiformen, die sich dadurch in Wirkungsdauer oder -geschwindigkeit verändern.

Der Ausdruck "Aminosäure" meint Verbindungen, welche mindestens eine Amino-und mindestens eine Carboxylgruppe enthalten. Obwohl die Aminogruppe üblicherweise in α-Position zur Carboxylgruppe steht, ist auch jede andere Anordnung im Molekül denkbar. Die Aminosäure kann auch weitere funktionelle Gruppen, wie z.B. Amino-, Carboxamid-, Carboxyl-, Imidazol-, Thiogruppen und andere Gruppen, enthalten. Verwendung finden Aminosäuren natürlichen oder synthetischen Ursprungs, razemisch oder optisch aktiv (D-, oder L-) inklusive verschiedener stereoisomerer Verhältnisse. Beispielsweise umfasst der Begriff Isoleucin sowohl D- Isoleucin, L- Isoleucin, razemisches Isoleucin und verschiedene Verhältnisse der beiden Enantiomere.

Mit dem Begriff "Peptid", "Polypeptid" oder "Protein" sind Polymere von Aminosäuren bestehend aus mehr als zwei Aminosäureresten gemeint. Weiterhin sind mit dem Begriff "Peptid", "Polypeptid" oder "Protein," Polymere von Aminosäuren bestehend aus mehr als 10 Aminosäureresten gemeint.

Der Begriff Peptid, Polypeptid oder Protein wird als pseudonym verwendet und umfasst sowohl Homo- als auch Heteropeptide, also Polymere von Aminosäuren bestehend aus identischen oder verschiedenen Aminosäureresten. Ein "DiPeptid" ist somit aus zwei peptidisch verknüpften Aminosäuren, ein "Tri-Peptid" aus drei peptidisch verknüpften Aminosäuren aufgebaut.

Der hier verwendete Begriff "Protein" meint Polymere von Aminosäuren mit mehr als 20 und insbesondere von mehr als 100 Aminosäureresten.

Der Begriff "kleines Protein" bezeichnet Proteine unter 50 kD bzw. unter 30 kD bzw. zwischen 5-50 kD. Der Begriff "kleines Protein" bezeichnet weiterhin Polymere von Aminosäureresten mit weniger als 500 Aminosäureresten bzw. von weniger als 300 Aminosäureresten bzw. von Polymeren mit 50-500 Aminosäureresten. Bevorzugte kleine Proteine sind z.B. Wachstumsfaktoren wie "human growth hormone/ factor", Insulin, Calcitonin oder Ähnliches.

Der Ausdruck "Oligosaccharid" oder "Polysaccharid" meint Mehrfachzucker die zumindest aus drei monomeren Zuckermolekülen aufgebaut sind.

Der Ausdruck "% (w/w)" meint den prozentualen, auf die Masse bezogenen Anteil eines Wirkstoffes bzw. eines Hilfsstoffes im sprühgetrockneten Pulver. Hierbei wird der angegebene Anteil auf die Trockensubstanz des Pulvers bezogen. Die Restfeuchte im Pulver wird demnach nicht mit berücksichtigt.

Der Begriff "amorph" meint, dass die pulverförmigen Formulierung weniger als 10 % kristalline Anteile enthalten, vorzugsweise weniger als 7 %, weiter bevorzugt weniger als 5 %, insbesondere weniger als 4, 3, 2, oder 1 %.

Der Begriff "inhalierbar" meint, dass die Pulver zur pulmonalen Applikation geeignet sind. Inhalierbare Pulver lassen sich mit.Hilfe eines Inhalationsgerätes dispergieren und inhalieren, so dass die Partikel die Lunge erreichen und gegebenenfalls über die Alveolen systemische Wirkung entfalten können. Inhalierbare Partikel weisen beispielsweise eine mittlere Teilchengröße zwischen 0,4-30 µm (MMD = Mass median diameter), meistens zwischen 0,5-20 µm, bevorzugt zwischen 1-10 µm und/oder einen mittleren aerodynamischen Teilchendurchmesser (MMAD = Mass median aerodynamic diameter) zwischen 0,5-10 µm, vorzugsweise zwischen 0,5-7,5 µm, weiter bevorzugt zwischen 0,5-5,5 µm, noch weiter bevorzugt 1-5 µm und in besonders bevorzugter Weise zwischen 1-4,5 µm bzw. 3-10 µm auf.

"Mass Median Diameter" oder "MMD" ist eine Messgröße für die durchschnittliche Partikelgrößenverteilung, da die Pulver der Erfindung generell polydispers sind. Die Ergebnisse werden ausgedrückt als Durchmesser der Volumensummenverteilung bei 50% Durchgangssumme. Die MMD-Werte lassen sich beispielhaft mittels Laserdiffraktometrie bestimmen, wobei natürlich auch jede andere übliche Methode verwendet werden kann (z.B. Elektronenmikroskopie, Zentrifugalsedimentation).

Der Begriff "mittlerer aerodynamischer Teilchendurchmesser" (= mass median aerodynamic diameter (MMAD)) gibt die aerodynamische Teilchengröße an, bei der normalerweise 50% der Teilchen bezogen auf die Masse des Pulvers einen kleineren aerodynamischen Durchmesser aufweisen. Als Referenzmethode zur Bestimmung des MMAD dient im Zweifel die in dieser Patentschrift angegebene Methode.

MMD und MMAD können voneinander abweichen, z.B. kann eine durch Sprühtrocknung entstandene Hohlkugel einen im Vergleich zum MMAD grösseren MMD aufweisen.

Der Begriff "*Feinpartikelfraktion*" (FPF) beschreibt den inhalierbaren Teil eines Pulvers bestehend aus Teilchen mit einer Teilchengröße von ≤ 5 µm MMAD. In gut dispergierbaren Pulvern beträgt die FPF mehr als 20%, vorzugsweise mehr als 30%, besonders bevorzugt mehr als 40%, noch weiter bevorzugt mehr als 50%, noch weiter bevorzugt mehr als 55%. Der in diesem Zusammenhang verwendete Begriff "Cut Off Diamenter" gibt an, welche Partikel bei der Bestimmung der FPF berücksichtigt werden. Eine FPF von 30% bei einem Cut Off Diameter von 5 µm (FPF₅) meint, dass zumindest 30% aller Partikel im Pulver einen mittleren aerodynamischen Teilchendurchmesser von kleiner 5 µm aufweisen.

Der Begriff " time of flight" ist die Bezeichnung für eine Standardmessmethode wie im Kapitel BEISPIELE näher beschrieben. Bei einer time of flight Messung wird der MMAD über die Bestimmung der Flugzeit eines Partikels für eine definierte Messstrecke ermittelt. Der MMAD korreliert mit der Flugzeit. Das heißt, dass Partikel mit einem großen MMAD eine größere Flugzeit benötigen als entsprechend kleiner Partikel. (vgl. hierzu: Kapitel BEISPIELE, Methode).

Der Begriff "ausgebrachte Masse" gibt die Pulvermenge an, die bei Anwendung eines Inhalators ausgebracht wird. Die Ausbringung wird in diesem Fall zum Beispiel anhand einer Kapsel bestimmt, indem die Kapsel vor und nach Ausbringung gewogen wird. Die ausgebrachte Masse entspricht der Massendifferenz der Kapsel vor und nach Ausbringung.

"Dispergierbar" meint flugtauglich. Grundvoraussetzung für eine Flugtauglichkeit eines Pulvers ist das Deagglomerieren des Pulvers in Einzelpartikel und das Verteilen der Einzelpartikel in Luft. Partikelagglomerate sind zu groß, um in die Lunge zu gelangen und sind demnach nicht geeignet für eine Inhalationstherapie.

Der Begriff "Raumtemperatur" bezeichnet eine Temperatur von ca. 20-25°C (+ /-10%). Der Begriff Raumtemperatur bezeichnet insbesondere eine Temperatur von 25°C.

Der Begriff "Monomergehalt" und "Monomer" bezeichnet den prozentualen Anteil an Proteinen bestehend aus einer einzigen Untereinheit des Proteins. Abzugrenzen vom Monomergehalt sind Fragmente bestehend aus Bruchstücken des Monomers und Di- bzw. Oligomere bestehend aus mehreren Untereinheiten. Der Monomergehalt wird beispielsweise mittels Ausschlusschromatographie bestimmt.

Der Begriff "Aggregate" meint den Anteil an Di- und Oligomeren von Proteinen, die im nativen Zustand aus einer einzigen Untereinheit bestehen.

### ERFINDUNGSGEMÄSSE ZUSAMMENSETZUNGEN

Die Faktoren, die das Flugverhalten der sprühgetrockneten Partikel (relevant ist hier die Feinpartikelfraktion FPF) beeinflussen sind die Grösse der Partikel (MMD bzw. insbesondere MMAD, welche mittels Time-of-Flight Messungen bestimmt wird) und das Dispergierverhalten der Pulver. Maßgeblich für das Dispergierverhalten der Pulver ist die chemische Zusammensetzung der Partikeloberfläche sowie die Morphologie der Partikel. Durch die gezielte Auswahl der Pulverbestandteile und insbesondere der Hilfsstoffe kann demnach das Dispergierverhalten der Pulver entscheidend beeinflusst werden.

Die Grösse und Morphologie eines Partikels ergibt sich bei der Trocknung eines einzelnen Tropfens nach Zerstäubung im Sprühtrockner folgendermassen:

Für die Herstellung inhalierbarer Pulver werden üblicherweise Zweistoffdüsen eingesetzt. Die Tröpfchengröße (MMD), die als Ausgangspunkt für die spätere Partikelgrösse relevant ist, liegt je nach Zerstäubergasrate bei ca. 8 -20µm. Die Trocknung des Tropfens erfolgt in 2 Schritten. In der ersten Phase verdampft Wasser, ohne dass Feststoff gebildet wird. Die Verdampfung ist nicht diffusionslimitiert. Nach Erreichen der Löslichkeitsgrenze eines der in der Lösung enthaltenen Stoffes kommt es zur Zweiphasenbildung fest/flüssig und es bildet sich schließlich eine geschlossene Feststoffschicht. Im Kern des werdenden Partikels sind weiterhin Wasser und gelöste Substanzen mit entsprechend höherer Löslichkeitsgrenze als die bereits ausgefallene Substanz enthalten.

Die zweite Phase der Partikelbildung beginnt nach Ausbildung der geschlossenen Feststoffschicht. Durch die Feststoffschicht wird die Verdampfungsrate des Wassers stark reduziert. Die Verdampfungsrate des Wassers hängt in der 2. Phase von der Diffusionsgeschwindigkeit des Wasser durch die Partikelschicht ab. Ist die Wasserdampfsdiffusion stark gehemmt, entsteht durch den Anstieg der Temperatur im Kern des werdenden Partikels ein erhöhter Dampfdruck. Um diesen auszugleichen, blähen sich die Partikel auf, wodurch Hohlkugeln entstehen. Nach dem Verdampfen des Wasser bzw. beim Abkühlen des Partikels entsteht im Kern des Partikels ein Unterdruck. Je nach Stabilität der Partikelschicht erstarrt das Partikel entweder in der aufgeblähten Form oder das Partikel kollabiert.

Die Neigung zum Kollabieren der Partikel hängt nicht nur von einer Stoff- bzw. Prozessgröße ab. Vielmehr ist es eine komplexe Funktion aus Hydrophobizität der Feststoffe, der erreichten Löslichkeitsgrenze und dem Feststoffanteil der Sprühlösung. Die Kombination aus Löslichkeitsgrenze und Feststoffanteil der Sprühlösung steuert des weiteren auch die Dicke der Partikelschicht. Weitere Einflußgrößen wie z.B. die Glasübergangstemperatur und daraus abgeleitet die Viskosität des Pulvers im Sprühtrockner könnten ebenfalls die Kollabierneigung beeinflussen.

Zusammenfassend kann festgehalten werden, dass im Groben die Neigung zum Aufblähen des werdenden Partikels mit der Hydrophobizität und mit abnehmender Löslichkeit der Hilfsstoffe zunimmt. Die Kollabierneigung der aufgeblähten Partikel scheint dagegen eine stoffspezifische Eigenschaft zu sein. Es konnte gezeigt werden, dass in diesem Zusammenhang Phenylalanin eine überraschend gute und unerwartete Morphologie des Pulvers bewirkt, insbesondere bei proteinhaltigen Pulvern und sprühgetrockneten Pulvern. Dieser Effekt ist besonders vorteilhaft für die Inhalation solcher Pulver.

Hilfsstoffe mit ähnlichen Hydrophobizitäten bzw. Löslichkeiten (Valin, Isoleucin) zeigten keine vergleichbare Morphologie und damit kein vergleichbares aerodynamisches Verhalten.

Die vorliegende Erfindung betrifft Pulver enthaltend ein Protein und Phenylalanin, **dadurch gekennzeichnet, dass** das Pulver mindestens 30% (w/w) Phenylalanin enthält (mindestens binärer Komplex).

Die Erfindung betrifft insbesondere ein Pulver enthaltend ein Protein, Phenylalanin und mindestens einen weiteren Hilfsstoff wie einen Zucker oder ein Polyol" **dadurch gekennzeichnet, dass** das Pulver mindestens 30% (w/w) Phenylalanin enthält (mindestens ternärer Komplex).

Die vorliegende Erfindung betrifft Pulver enthaltend ein Protein und Phenylalanin, **dadurch gekennzeichnet, dass** das Pulver mindestens 40% (w/w) Phenylalanin enthält (mindestens binärer Komplex).

Die Erfindung betrifft insbesondere ein Pulver enthaltend ein Protein, Phenylalanin und mindestens einen weiteren Hilfsstoff wie einen Zucker oder ein Polyol" **dadurch gekennzeichnet, dass** das Pulver mindestens 40% (w/w) Phenylalanin enthält (mindestens ternärer Komplex).

In einer bevorzugten Ausführungsform ist das vorliegende Pulver (mindestens binär oder mindestens ternär) ein sprühgetrocknetes Pulver.

In einer speziellen Ausführungsform betrifft die Erfindung Pulver enthaltend einen Protein oder einen Protein-Wirkstoff und Phenylalanin als Hilfsstoff sowie optional einen weiteren Hilfsstoff wie einen Zucker oder ein Polyol , wobei das Pulver dadurch gekennzeichnet ist, dass es mindestens 30% (w/w) Phenylalanin, bevorzugt mindestens 40% (w/w) Phenylalanin enthält. Optional können auch weitere Substanzen insbesondere weitere Hilfsstoffe im Pulver enthalten sein. Weiterhin betrifft diese spezielle Ausführungsform der vorliegenden Erfindung auch eine pharmazeutische Zusammensetzung, welche ein Pulver, bestehend aus einen Protein oder einem Protein-Wirkstoff und Phenylalanin als Hilfsstoff sowie optional einem weiteren Hilfsstoff wie einen Zucker oder ein Polyol , enthält, wobei das Pulver zu mindestens 30% (w/w) aus Phenylalanin, bevorzugt mindestens 40% (w/w) aus Phenylalanin besteht.

In einer weiteren Ausführungsform enthält das vorliegende Pulver mindestens 30% (w/w), 31% (w/w), 32 (w/w), 33 (w/w), 34 (w/w), 35 (w/w), 36 (w/w), 37 (w/w), 38 (w/w), 39 (w/w), 40% (w/w), 41% (w/w), 42% (w/w), 43% (w/w), 44% (w/w), 45% (w/w), 46% (w/w), 47% (w/w), 48% (w/w), 49% (w/w), 50% (w/w), 51 % (w/w), 52% (w/w), 53% (w/w), 54% (w/w), 55% (w/w), 56% (w/w), 57% (w/w), 58% (w/w), 59% (w/w), 60% (w/w), 61 % (w/w), 62% (w/w), 63% (w/w), 64% (w/w), 65% (w/w), 66% (w/w), 67% (w/w), 68% (w/w), 69% (w/w), 70% (w/w), 75% (w/w), 80% (w/w), 85% (w/w), 90% (w/w), 95% (w/w), 99% (w/w) oder 99.99% (w/w) Phenylalanin. Hohe prozentuale Anteile an Phenylalanin sind insbesondere bei hochpotenten Proteinen wie Zytokinen und Interferonen (IFN-alpha, IFN-beta, IFN-gamma, IFNomega, pegyliertes IFN etc. ) bevorzugt, da von diesem Protein nur geringe Mengen benötigt werden (0.01% (w/w) bis 10% (w/w), insbesondere 0.01% (w/w) bis 5% (w/w) und insbesondere 0.01 % (w/w) bis 1 % (w/w)).

In einer bevorzugten Ausführungsform enthält das vorliegende Pulver einen Anteil an Phenylalanin im Bereich von 30% (w/w) bis 99.99% (w/w), bevorzugt 40% (w/w) bis 99.99% (w/w) , bevorzugt 40% (w/w) bis 70% (w/w), 60% - 90% oder besonders bevorzugt 60% bis 80%.

In einer weiteren Ausführungsform enthält das vorliegende Pulver einen nicht reduzierender Zucker ausgewählt aus der Gruppe bestehend aus einem Disaccharid und einem Oligosaccharid. Bevorzugterweise ist das Disaccharid Saccharose oder Trehalose, das Oligosaccharid ein Trisaccharid wie beispielsweise Lactosucrose.

In einer weiteren Ausführungsform beträgt der Zuckeranteil maximal 50% (w/w), bevorzugt 5, 10, 15, 20, 25, 30, 35, 40, 45% (w/w) und besonders bevorzugt 10 bis 20% (w/w).

In einer weiteren Ausführungsform enthält das vorliegende Pulver ein Polyol. Bevorzugterweise ist das Polyol Mannitol.

In einer weiteren Ausführungsform beträgt das Massenverhältnis Zucker zu Protein zwischen 1:10 bis 10:1, bevorzugt 1:3 bis 5:1.

In einer weiteren bevorzugten Ausführungsform enthält das vorliegende Pulver einen Kristallisationsinhibitor wie HSA (humanes Serum Albumin) Bevorzugt enthält das Pulver mindestens 0.1% (w/w) HSA, mindestens 0.5%(w/w) HSA, mindestens 1% (w/w) HSA, mindestens 5%(w/w) HSA, mindestens 10%(w/w) HSA, mindestens 15% (w/w) HSA. Weiterhin bevorzugt enthält das Pulver zwischen 0.1 %(w/w) - 60%(w/w) HSA, 0.5% (w/w) - 60% (w/w)HSA, 1 % (w/w) - 60% (w/w) HSA, 10% (w/w) - 60% (w/w) HSA, 0.1 %(w/w) - 40%(w/w) HSA, 0.5% (w/w) - 40% (w/w)HSA, 1 % (w/w) - 40% (w/w) HSA, 10% (w/w) - 40% (w/w) HSA, 0.1 %(w/w) - 20%(w/w) HSA, 0.5% (w/w) - 20% (w/w)HSA, 1 % (w/w) - 20% (w/w) HSA, 10% (w/w) - 20% (w/w) HSA, 0.1 %(w/w) - 1 %(w/w) HSA, 0.5% (w/w) - 1 % (w/w)HSA, 0.1% (w/w) - 0.90% (w/w) HSA, 0.5% (w/w) - 0.9% (w/w) HSA, 0.1 %(w/w) - 3%(w/w) HSA, 0.5 %(w/w) - 3%(w/w) HSA. Weiterhin bevorzugt enthält das Pulver weniger als 1 % (w/w) HSA, weniger als 0.9% (w/w) HSA .

In einer weiteren bevorzugten Ausführungsform liegt das vorliegende Pulver bei einem ph-Wert > 6.0, >6.5, >7.0, >7.4, >8 vor. Insbesondere bevorzugt ist ein pH-Bereich zwischen 6.0 bis 9.0 bzw. 7.0 bis 8.0.

In einer weiteren besonders bevorzugten Ausführungsform liegt das vorliegende Pulver bei physiologischem pH-Wert vor. In einer weiteren besonders bevorzugten Ausführungsform liegt das vorliegende Pulver bei pH 7.0 bis 7.4 vor. In einer weiteren bevorzugten Ausführungsform liegt das vorliegende Pulver bei einem pH-Wert, der nicht dem isoelektrischen Punkt von Phenylalanin entspricht.

In einer bevorzugten Ausführungsform ist das Protein ein Wirkstoff, bevorzugt ein pharmazeutischer Wirkstoff wie beispielsweise ein Antikörper, ein Antikörperfragment, ein Fusionsprotein mit Teilen von Antikörpern oder ein konjugierter Antikörper, ein Wachstumsfaktor, ein Hormon, ein Enzym, ein Zytokin oder ein Interferon. In einer besonders bevorzugten Ausführungsform ist der pharmazeutische Wirkstoff Insulin oder Calcitonin.

In einer weiteren Aufführungsform ist der pharmazeutische Wirkstoff ein Fusionsprotein oder eine Antikörperfragment, der an den neonatalen Fc-Rezeptor bindet.

In einer weiteren Ausführungsform beträgt der Proteingehalt 0,01-70% (w/w), 0,01-60% (w/w), 0,01-50% (w/w), 0,01-40% (w/w), 1-50% (w/w), 10-50% (w/w) und bevorzugt 30-50% (w/w).

In einer bevorzugten Ausführungsform beträgt das Verhältnis Phenylalanin /Zucker / Protein 40/10/50, 99.89/0.1/0.01, 90/9/1, 90/1/9, 80/10/10, 30/10/60, bevorzugt 60/10/30 oder 50/10/40.

In einer besonders bevorzugten Ausführungsform besteht das Pulver aus Phenylalanin / Lactosucrose oder Saccharose / und einem kleinen Protein im Massenverhältnis 60/10/30.

In einer weiteren Ausführungsform ist die mittlere aerodynamische Teilchengröße (MMAD = Mass median aerodynamic diameter) der Pulverpartikel kleiner 10µm, bevorzugt kleiner 7,5, noch bevorzugt im Bereich zwischen 1-6µm bzw. 3-6µm oder 5-7µm.

Die Erfindung betrifft in einer weiteren Ausführungsform eine pharmazeutische Zusammensetzung, die das erfindungsgemäße Pulver enthält.

In einer weiteren Ausführungsform enthält die pharmazeutische Zusammensetzung zudem pharmazeutisch verträgliche Hilfsstoffe bzw. pharmazeutisch akzeptable Hilfsstoffe wie pharmazeutisch akzeptable Salze, Puffer, Detergentien und dergleichen.

Die vorliegende Erfindung betrifft zudem ein Verfahren zur Herstellung eines erfindungsgemäßen Pulvers, wobei
a) eine Phenylalaninlösung hergestellt wird,
b) mindestens ein Protein und optional mindestens ein weiterer Hilfsstoff wie ein Zucker oder ein Polyol hinzugefügt werden,
c) die so erhaltene Lösung oder Suspension bei einer Einströmtemperatur von vorzugsweise 90 - 200°C und einer Ausströmtemperatur von vorzugsweise 40 - 150°C versprüht wird und
d) die entstandenen Partikel vom Trocknungsgas abgetrennt werden.

In einer bevorzugten Ausführungsform des erfindungsgemäßen Verfahrens ist das Lösungsmittel Wasser, Ethanol, Isopropanol etc..

In einer besonders bevorzugten Ausführungsform des vorliegenen Verfahrens handelt es sich bei dem Protein um einen pharmazeutischen Wirkstoff. Der pharmazeutischen Wirkstoff ist bevorzugt ein kleines Protein, ein Antikörper, ein Antikörperfragment, ein Fusionsprotein mit Teilen von Antikörpern oder ein konjugierter Antikörper, ein Wachstumsfaktor, ein Hormon, ein Enzym, ein Zytokin oder ein Interferon. In einer besonders bevorzugten Ausführungsform ist der pharmazeutische Wirkstoff Insulin, Calcitonin. In einer weiteren ganz besonders bevorzugten Ausführungsform ist der pharmazeutische Wirkstoff ein Antikörper der Klasse IgG1, IgG2 IgG3, IgG4, ein Antikörperfragment, ein Interferon oder Ähnliches.

In einer weiteren bevorzugten Ausführungsform des vorliegenden Verfahren wird im Schritt b) zunächst der weitere Hilfsstoff wie ein Zucker oder ein Polyol hinzugefügt und danach der Wirkstoff.

In einer weiteren Ausführungsform des vorliegenden Verfahrens werden zwischen dem Schritt a) und b) folgende weitere Schritte durchgeführt
- eine Erwärmung der Phenylalaninlösung, vorzugsweise auf 80°C,
- eine Abkühlung der Phenylalaninlösung bis unterhalb der Denaturierungstemperatur des jeweils hinzuzufügenden Proteins, wobei die Abkühlung bevorzugt bis auf Raumtememperatur erfolgt.

In einer bevorzugten Ausführungsform des vorliegenden Verfahrens wird die Lösung oder Suspension im Schritt c) mittels mindestens einer Druckdüse oder mindestens eines Rotationszerstäubers oder mindestens einer Venturidüse oder mindestens eines Ultraschallverneblers oder mindestens einer Zweistoffdüse versprüht. In einer besonders bevorzugen Ausführungsform wird die Lösung oder Suspension im Schritt c) mittels mindestens einer Zweistoffdüse versprüht.

In einer weiteren bevorzugten Ausführungsform des vorliegenden Verfahrens erfolgt die Abtrennung der Partikel in Schritt d) mittels mindestens eines Partikelabscheiders, bevorzugt mittels mindestens eines Zyklons.

Die vorliegende Erfindung betrifft weiterhin die Verwendung eines erfindungsgemäßen Pulvers oder einer erfindungsgemäßen pharmazeutischen Zusammensetzung als Arzneimittel (1. medizinische Indikation).

In einer bevorzugten medizinischen Verwendung enthält das Arzneimitel ein erfindungsgemäßes sprühgetrocknetes Pulver.

Die vorliegende Erfindung betrifft weiterhin die Verwendung eines erfindungsgemäßen Pulvers oder einer erfindungsgemäßen pharmazeutischen Zusammensetzung als inhalatives Arzneimittel.

In einer bevorzugten medizinischen Verwendung enthält das inhalative Arzneimittel ein erfindungsgemäßes sprühgetrocknetes Pulver.

Die Erfindung betrifft weiterhin die Verwendung eines erfindungsgemäßen Pulvers oder einer erfindungsgemäßen pharmazeutischen Zusammensetzung zur Herstellung eines Medikaments zur Behandlung von Atemwegserkrankungen oder systemischen Erkrankungen (2. med. Indikation).

In einer bevorzugten Ausführungsform ist das zur Herstellung eines Medikaments zur Behandlung von Atemwegserkrankungen oder systemischen Erkrankungen verwendete erfindungsgemäße Pulver oder die verwendete erfindungsgemäße pharmazeutische Zusammensetzung sprühgetrocknet.

In einer besonders bevorzugten Ausführungsform ist die Atemwegserkrankung oder systemische Erkrankung ausgewählt aus der Gruppe bestehen aus Lungenkrebs, Lungenentzündung, cystische Fibrose, COPD (chronisch obstruktive Lungenerkrankung), Asthma, anti-inflammatorische Erkrankungen, virale Erkrankungen z.B. durch den respiratorisch-syncytial Virus (RSV).

Eine bevorzugte Ausführungsform der vorliegenden Erfindung bezieht sich auf ein erfinderisches Pulver bevorzugt ein sprühgetrocknetes Pulver, wobei dieses Pulver keinen Zusatz von Magnesiumstearat enthält. Für das Hydrophobisieren von Partikeloberflächen durch Sprühtrocknung ist Magnesiumstearat nicht geeignet, da diese Substanz praktisch in Wasser unlöslich ist und dementsprechend Magnesiumstearat-Suspensionen eingesetzt werden müssten. In diesem Fall sind verhältnismäßig hohe Magnesiumstearat Konzentrationen notwendig, um die gewünschte Partikelbelegung zu gewährleisten. Geeignetere Verfahren sind daher separate Prozessschritte, z.B. das Mischen des (sprühgetrockneten) Pulvers mit Magnesiumstearat.

In einer weiteren bevorzugten Ausführungsform enthält das erfinderische Pulver, welches bevorzugt sprühgetrocknet ist, oder die erfinderische pharmazeutische Zusammensetzung keine weiteren Aminosäuren außer Phenylalanin. Das (sprühgetrocknete) Pulver enthält also bevorzugt ausschließlich die Aminosäure Phenylalanin. Diese Ausführungsform ist bevorzugt, da andere Aminosäuren, den überraschenden aerodynamischen Effekt des Phenylalanins vermindern bzw. ausdünnen.

Eine weitere bevorzugte Ausführungsform der vorliegenden Erfindung bezieht sich auf ein erfinderisches Pulver bevorzugt ein sprühgetrocknetes Pulver, wobei dieses Pulver keinen Zusatz von Valin enthält. Das bevorzugte Pulver ist frei von Valin.

Eine weitere bevorzugte Ausführungsform der vorliegenden Erfindung bezieht sich auf ein erfinderisches Pulver bevorzugt ein sprühgetrocknetes Pulver, wobei dieses Pulver keinen Zusatz von Isoleucin enthält. Das bevorzugte Pulver ist frei von Isoleucin.

Eine weitere bevorzugte Ausführungsform der vorliegenden Erfindung bezieht sich auf ein erfinderisches Pulver bevorzugt ein sprühgetrocknetes Pulver, wobei dieses Pulver keinen Zusatz von Leucin enthält. Das bevorzugte Pulver ist frei von Leucin.

In einer weiteren bevorzugten Ausführungsform enthält das Pulver, welches bevorzugt sprühgetrocknet ist, keinen Zusatz von Tensiden wie Tween 20. Diese Ausführungsform ist bevorzugt, da Tenside eher destabilisierend auf Proteinpulver, insbesondere sprühgetrocknete Proteinpulver wirken.

Eine weitere bevorzugte Ausführungsform der vorliegenden Erfindung bezieht sich auf ein erfinderisches Pulver bevorzugt ein sprühgetrocknetes Pulver, wobei dieses Pulver keinen Zusatz von Dextran enthält. Das bevorzugte Pulver ist frei von Dextran. Dextran-haltige Pulver weisen eine verschlechterte Dispergierbarkeit auf und sind daher weniger bevorzugt.

Aus den nachfolgenden Experimenten wird ersichtlich, dass die hydrophoberen Aminosäuren ein Aufblähen der Partikel verursachen. Die Kollabierneigung in Abhängigkeit von der enthaltenen Aminosäure ist hingegen nicht vorhersagbar und folgt keiner strukturell begründeten Gesetzmässigkeit. In den nachfolgenden Beispielen nimmt die Kollabierneigung in der Reihe Valin, Isoleucin, Phenylalanin überraschend zu. Während Valin runde Partikel ausbildet, sind Phenylalanin-haltige Partikel annähernd komplett kollabiert. Das Phenylalanin-haltige Pulver hat überraschenderweise extrem gute aerodynamische Eigenschaften. Es können unabhängig vom Sättigungsgrad der Aminosäure Feinpartikel Fraktionen (FPF) von 65-72% realisiert werden.

Hervorzuheben ist außerdem, dass die maximal erreichte FPF bei den Phenylalanin-haltigen Pulvern verglichen zu Pulvern, insbesondere sprühgetrockneten Pulvern, die nicht Phenylalanin sondern andere Hilfsstoffe enthalten, sehr hoch ist. Die maximal erreichbare FPF ergibt sich aus dem Vergleich der FPF ermittelt über die Impaktorstufe und dem über die Flugzeitbestimmung ermittelten Anteil kleiner 5 µm. Hiernach ergibt sich für gut dispergierbare Pulver nur eine geringe Diskrepanz zwischen der FPF der Impaktorstufe und der über die Flugzeitbestimmung ermittelten Fraktion <5 µm. Bei schlecht dispergierbaren Pulver zeigt sich hingegen, dass die über die Impaktorstufe erzielte FPF wesentlich kleiner ist. Ursache ist, dass beim Impaktorverfahren die Feinpartikelfraktion über sämtliche Fraktionen ermittelt wird. Das heißt, dass die Verluste durch verbleibendes Pulver z.B. in der Kapsel, im Inhalator sowie im Sample Induction Port die ermittelte FPF reduziert. Bei der Flugzeitbestimmung wird hingegen nur über das bereits dispergierte Pulver bilanziert, was bedeutet, dass die oben genannten Verluste nicht in die Messung eingehen.

Es ist anzunehmen, dass das aerodynamische Verhalten der Partikel stark von der Partikelmorphologie und der Oberflächenbeschaffenheit abhängt. Demnach sind Mehrfachdellen der Partikel bzw. stark kollabierte Partikel, wie bei Phenylalanin-haltigen Partikeln festgestellt, für die Inhalation ideal.

Durch das Kollabieren und der damit verbundenen ungleichmäßigen Form werden Van-der-Waals Kräfte abgeschwächt. Zusätzlich zeigen die Phenylalanin-haltigen Partikel im Gegensatz zu den Valin- und Isoleucin-haltigen Partikeln eine wesentlich rauere Oberflächenstruktur. Die raue Oberflächenstruktur könnte durch eine Kristallisation verursacht worden sein.

In den nachfolgenden Beispielen konnte gezeigt werden, dass Phenylalanin alleine und im besonderen in Kombination mit einem Zucker sehr gute aerodynamische Eigenschaften von Pulver, insbesondere nach Sprühtrocknung, hervorbringt. Phenylalanin allein vermag jedoch nicht jedes Protein, z.B. den in den Beispielen 1 und 2 verwendeten IgG1-Antikörper, zu stabilisieren. Für solche Proteine ist jedoch eine Stabilisierung durch Zugabe von Zucker möglich.

Die Beispiele verdeutlichen, dass das Protein unter trockenen Lagerbedingungen sowohl bei 25°C als auch bei 40°C über die geprüfte Lagerzeit von 1 Monat, 2 Monaten und 3 Monaten annähernd vollständig stabilisiert gelagert werden kann. Unter feuchten Bedingungen kann es zu einer leichten Schädigung des Proteins wie dem im Beispiel verwendeten Antikörpers kommen.

Die nachfolgenden Beispiele verdeutlichen weiterhin, dass das Phenylalanin-haltige Pulver verglichen zu einem Dextran-haltigen Pulver eine wesentlich bessere FPF hat (59,6% vers. 33,7%). Da die aerodynamischen Teilchengrößen der beiden Pulver nur geringfügig unterschiedlich sind bzw. das Phenylalanin-haltige Pulver sogar einen leicht erhöhten MMAD aufweist, sind die Unterschiede der FPF auf das Dispergierverhalten der Pulver beim Ausbringen aus der Kapsel zurück zu führen. Das bedeutet, dass sich das Phenylalanin-haltige Pulver wesentlich besser dispergieren lässt und somit interpartikuläre Wechselwirkungen geringer sind, verglichen zum entsprechenden Dextran-haltigen Pulver. In den Beispielen zeigt sich weiterhin, dass das Phenylalanin-haltige Pulver im Vergleich zu eine Dextran-haltigen Pulver über die Lagerzeit wesentlich geringere Einbrüche in der FPF aufweist. Besonders vorteilhaft ist das Phenylalanin bei höheren Luftfeuchten (z.B. 25°C/60% relative Feuchte). Während beim Dextran-haltigen Pulver die FPF auf 45-49% des Ausgangswertes abfällt, zeigt das Phenylalanin-haltige Pulver nach 2 Monaten Lagerung bei 25°C/60% relative Feuchte sogar einen Anstieg in der FPF und nach 3 Monaten nur einen leichten Abfall auf 89% des Ausgangswertes.

Die Ergebnisse der Beispiele unterstreichen im Besonderen die Eignung der ternären Pulver-zusammensetzungen bei erhöhten Luftfeuchten. Die herkömmlichen Pulver, insbesondere sprühgetrockneten Pulver, zeigen bei Aussetzung von erhöhten Luftfeuchten in der Regel einen starken Einbruch im aerodynamischen Verhalten. Durch das Phenylalanin kommt es dagegen bei einer Lagerung bei hohen Luftfeuchten (z.B. 60% relative Feuchte) zu einer Stabilisierung der Aerodynamik bzw., wie in den Beispielen gezeigt, zu einer Verbesserung derselben.

### Morphologie der Pulver:

Wie die nachfolgenden Beispiele verdeutlichen, zeigen beide Pulver, sowohl Phenylalanin-haltiges Pulver als auch Dextran-haltiges Pulver keine größeren Pulveragglomerate. Des weiteren sind bei den Formulierungen Mehrfachdellen zu sehen. Ein wesentlicher Unterschied in den beiden Morphologien ist die höhere Oberflächenrauigkeit beim Phenylalanin-haltigen Pulver. Diese erhöhte Oberflächenrauigkeit ist vermutlich auch der Grund für ein besseres Dispergierverhalten.

### BEISPIELE

### BEISPIEL 1 BINÄRE KOMPLEXE

Es wurden binäre Lösungen aus einem IgG1 und verschiedenen Aminosäuren, die sich in Löslichkeit und Hydrophobizität unterscheiden, hergestellt. Die Konzentration an Aminosäure in der Sprühlösung lag bei den eingesetzten Aminosäuren bei 50% und in einer weiteren Versuchsreihe bei 90% der maximal erreichbaren Konzentration der jeweiligen Aminosäure (siehe Tabelle 1). Das Massenverhältnis zwischen IgG1 und Aminosäure war 95 / 5. Bedingt durch die unterschiedlichen Löslichkeiten der Aminosäuren stellten sich entsprechend unterschiedliche Feststoffanteile ein.

**Tabelle 1: binäre Lösungen aus IgG1 und Hilfsstoff**

| **Charge** | **Protein** | **Hilfsstoff** | **Sättigungsgrad AS** | **Feststoffanteil, %** |
|---|---|---|---|---|
| 1 | IgG1 | Isoleucin | 90 % | 3,5 |
| 2 | IgG 1 | Glycin | 90 % | 20,2 |
| 3 | IgG 1 | Valin | 90 % | 5,8 |
| 4 | IgG 1 | Phenylalanin | 90 % | 3,2 |
| 5 | IgG 1 | Asparagin | 90 % | 2,4 |
| 6 | IgG 1 | Glycin | 50% | 11,18 |
| 7 | IgG 1 | Isoleucin | 50 % | 1,95 |
| 8 | IaG 1 | Valin | 50 % | 3,21 |
| 9 | IgG 1 | Phenylalanin | 50 % | 1,79 |
| 10 | IgG1 | Asparagin | 50 % | 1,3 |

Die Lösungen wurden unter folgenden Sprühbedingungen sprühgetrocknet:
- Sprühtrockner:: SD-Micro (Fa. Niro)
- Engangstemperatur: 120 °C
- Ausgangstemperatur:: 90 °C
- Zerstäubergasrate:: 5 kg/h
- Trocknungsgasrate:: 28 kg/h

Es zeigte sich, dass die hydrophoberen Aminosäuren ein Aufblähen der Partikel verursachen. Die Kollabierneigung nahm in der Reihe Valin, Isoleucin und Phenylalanin zu. Während Valin runde Partikel ausbildete, war Phenylalanin annähernd komplett kollabiert (siehe Abbildung 1a-1c). Das Phenylalanin-haltige Pulver hatte überraschenderweise extrem gute aerodynamische Eigenschaften. Es konnten unabhängig vom Sättigungsgrad der Aminosäure Feinpartikel Fraktionen (FPF) von 65-72 % realisiert werden (siehe Tabelle 2).

Hervorzuheben ist außerdem, dass die maximal erreichte FPF bei den Phenylalanin-haltigen Pulvern verglichen zu sprühgetrockneten Pulven mit den in Tabelle 1 gelisteten Hilfsstoffen sehr hoch ist. Die maximal erreichbare FPF ergibt sich aus dem Vergleich der FPF ermittelt über die Impaktorstufe und dem über die Flugzeitbestimmung ermittelten Anteil kleiner 5 µm. Die APS-Methode wird in der Beschreibung zu Abbildung 5/6 detailliert erläutert. Hiernach ergibt sich für gut dispergierbare Pulver nur eine geringe Diskrepanz zwischen der FPF der Impaktorstufe und der über die Flugzeitbestimmung ermittelten Fraktion <5µm. Bei schlecht dispergierbaren Pulver zeigt sich hingegen, dass die über die Impaktorstufe erzielte FPF wesentlich kleiner ist. Ursache ist, dass beim Impaktorverfahren die Feinpartikelfraktion über sämtliche Fraktionen ermittelt wird. Das heißt, dass die Verluste durch verbleibendes Pulver z.B. in der Kapsel, im Inhalator sowie im Sample Induction Port die ermittelte FPF reduziert. Bei der Flugzeitbestimmung wird hingegen nur über das bereits dispergierte Pulver bilanziert, was bedeutet, dass die oben genannten Verluste nicht in die Messung eingehen.

Es ist anzunehmen, dass das aerodynamische Verhalten der Partikel stark von der Partikelmorphologie und der Oberflächenbeschaffenheit abhängt. Demnach sind Mehrfachdellen der Partikel bzw. stark kollabierte Partikel, wie bei Phenylalanin-haltigen Partikeln festgestellt, für die Inhalation ideal.

Durch das Kollabieren und der damit verbundenen ungleichmäßigen Form werden Van-der-Waals Kräfte abgeschwächt. Zusätzlich zeigten die Phenylalanin-haltigen Partikel im Gegensatz zu den Valin- und Isoleucin-haltigen Partikeln eine wesentlich rauere Oberflächenstruktur. Die raue Oberflächenstruktur könnte durch eine Kristallisation verursacht worden sein.

**Tabelle 2: Aerodynamische Verhalten der sprühgetrockneten Pulver, gemessen mit dem Aerodynamic Particle Sizer mit Impactor Inlet**

| **Hilfsstoff** | **MMAD^{a}** | **Anteil Partikel < 5,0µm, % ^{a}** | **FPF, % ^{b}** | **Erreichungsgrad der max. FPF, % ^{c}** |
|---|---|---|---|---|
| Glycin (90%) | 4,94 | 55 | 24,6 | 45 |
| Glycin (50%) | 4,24 | 70 | 32,7 | 47 |
| Isoleucin (90%) | 2,06 | 89 | 59 | 66 |
| Isoleucin (50%) | 1,97 | 86 | 55,1 | 64 |
| Asparagin (90%) | 2,76 | 94 | 28,7 | 31 |
| Asparagin (50%) | 2,77 | 87 | 14,7 | 17 |
| Valin (90%) | 2,38 | 96 | 27,4 | 29 |
| Valin (50%) | 2,26 | 98 | 28,4 | 29 |
| Phenylalanin (90%) | 2,85 | 82 | 64,6 | 79 |
| Phenylalanin (50%) | 2,6 | 91 | 71,8 | 79 |

| | | | | |
|---|---|---|---|---|
| ^{a} der MMAD wurde über eine Time-of-Flight Messung (TOF) ermittelt. Hierzu wird das Pulver mit dem HandiHaler bei einer Flussrate von 39,0 L/min über einen Sample Induction Port (SIP) ausgebracht. Nach dem Passieren des SIP wird das Pulver-Aerosol gesplittet. Ein Anteil von 99,8 % der Partikelpopulation wird über einen Einstufenimpaktor geleitet,. Ein Anteil von 0,2 % geht über eine Kapillare in die TOF-Messzelle. ^{b} die FPF wird mit einem Einstufenimpaktor ermittelt. Der Cut-off der Impaktorstufe lieft bei 5,0 µm bei einer Flussrate von 39,0 L/min. ^{c} Die maximal erreichbare FPF ist gleich dem in der TOF-Messzelle ermittelten Anteil <5µm. In der TOF-Messzelle wird das Pulver-Aerosol vermessen, das unmittelbar über die Impaktorstufe geleitet wird. Die TOF-Messung hat demnach keinen Bezug zu Partikelfraktionen, die sich zuvor in der Messeinrichtung (Kapsel, HandiHaler, SIP) abgeschieden haben. Die FPF bezieht sich dagegen auf die Einwaage in der Kapsel. Hier gehen demnach Partikelfraktionen ein, die vor dem Erreichen der Impaktorstufe abgeschieden wurden. Ist die FPF gleich dem in der TOF-Messzelle ermittelten Partikelanteil < 5,0 µm, so wurde folglich das Pulver komplett dispergiert und es liegen keine Pulverablagerungen in HandiHaler und SIP vor. | | | | |

In Abbildung 2 ist der Monomerengehalt des Antikörpers nach Sprühtrocknung dargestellt. Hieraus ist ersichtlich, dass die weniger hydrophoben Aminosäuren (Glycin, Asparagin) auf den Antikörper stabilisierend wirken. Die hydrophoben Aminosäuren (Valin, Isoleucin und Phenylalanin zeigten dagegen kein ausreichend stabilisierendes Potential für den Antikörper.

### BEISPIEL 2 TERNÄRE KOMPLEXE

Basierend auf Beispiel 1 wurden ternäre Gemische aus IgG1, Phenylalanin und einem weiteren Hilfsstoff hergestellt. Bei der 3. Komponente handelte es sich um das sehr gut wasserlösliche Trisaccharid Lactosucrose LS90P.

Es wurden 4 Sprühlösungen hergestellt (siehe Tabelle 3). Das Lösungsmittel war gereinigtes Wasser. Der Feststoffanteil in der Sprühlösung lag jeweils bei 3,83% (w/v).

**Tabelle 3: ternäre Pulverzusammensetzungen aus Phenylalanin, Zucker und Protein**

| | **Prozentuale Zusammensetzung im Pulver (Phenylalanin/LS90P/ Protein)** | **Protein/Zucker Massenverhältnis** |
|---|---|---|
| Pulver 1 | 80/10/10 | 1:1 |
| Pulver 2 | 80/15/5 | 1:3 |
| Pulver 3 | 60/30/10 | 1:3 |
| Pulver 4 | 70/25/5 | 1:5 |

Die Lösungen wurden unter folgenden Sprühbedingungen sprühgetrocknet:
- Sprühtrockner:: SD-Micro (Fa. Niro)
- Engangstemperatur: 120 °C
- Ausgangstemperatur:: 90 °C
- Zerstäubergasrate:: 4 kg/h
- Trocknungsgasrate:: 28 kg/h

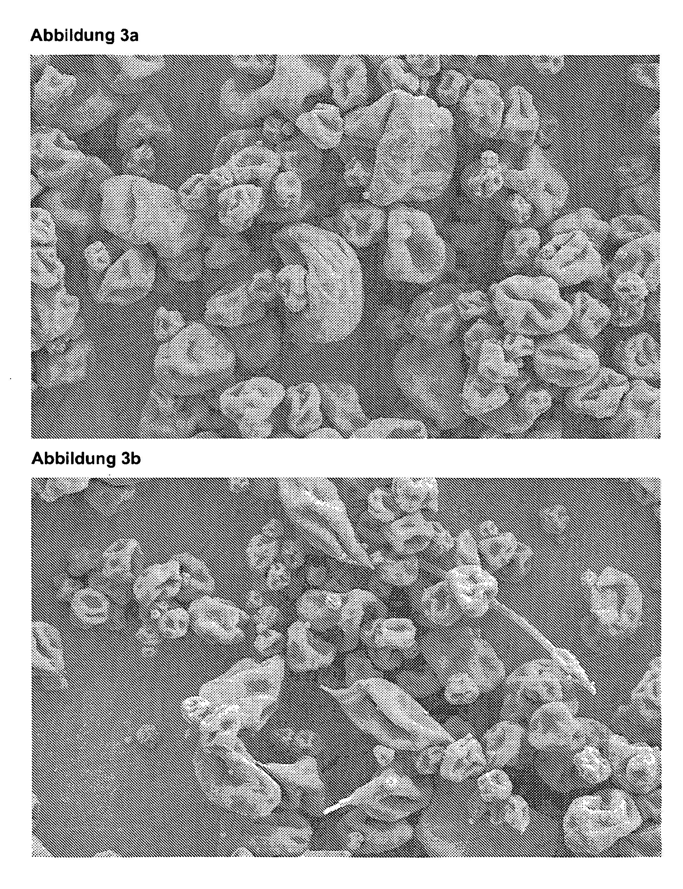

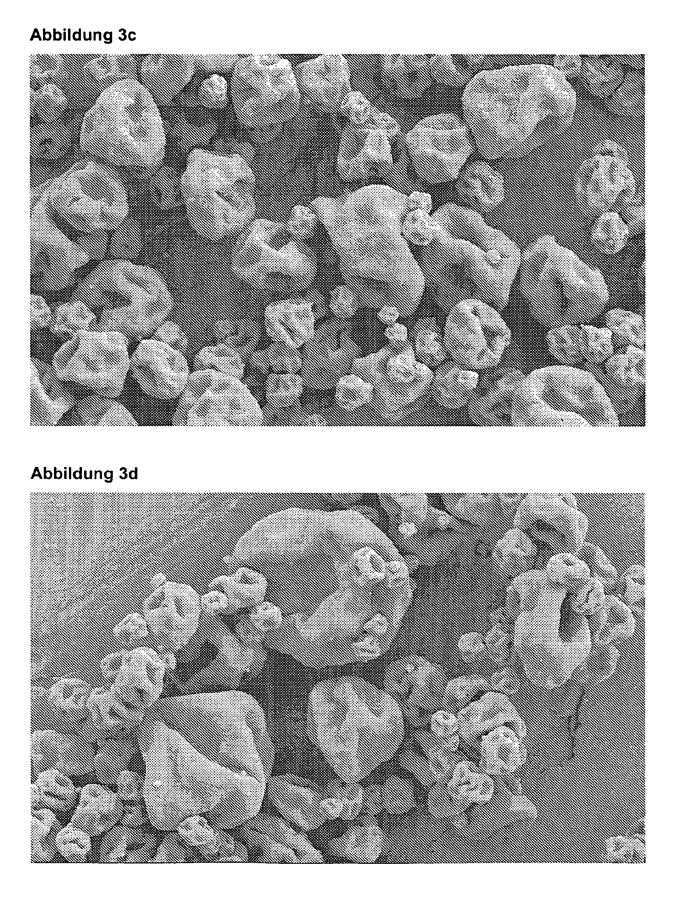

Die Abbildungen 3a-3d zeigen die REM-Aufnahmen der verschiedenen ternären Pulver. Die 4 Pulver zeigen die gleiche Faltung wie die Pulverkomposition aus Phenylalanin und IgG1 (siehe Beispiel 1). Die 4 ternären Pulver zeigen untereinander keine signifikanten Unterschiede.

In Tabelle 4 sind die aerodynamischen Eigenschaften der 4 Pulver aufgelistet. Durch die Zugabe von Laktosucrose nimmt die FPF verglichen zu den binären Zusammensetzungen nur geringfügig ab. Die Proteinstabilisierung nach Sprühtrocknung der ternären Pulverkompositionen ist dagegen sehr gut. Der Monomergehalt lag bei allen Formulierungen zwischen 98-99 % (siehe Tabelle 5)

**Tabelle 4: Aerodynamische Verhalten der sprühgetrockneten Pulver, gemessen mit dem APS***

| **Formulierung** **Phenylalanin/ LS90P/ IgG 1** | **MMAD [µm]** | **FPF [%]** |
|---|---|---|
| 80/10/10 | 3,03 | 55,7 |
| 80/15/5 | 2,91 | 64,3 |
| 60/30/10 | 3,35 | 48,4 |
| 70/25/5 | 3,39 | 55,8 |

| | | |
|---|---|---|
| * Die Messungen erfolgten mit dem Aerodynamic Particle Sizer. | | |

**Tabelle 5: Monomerengehalt der ternären Pulverzusammensetzungen**

| **Prozentuale Zusammensetzung des Pulvers** | | **Monomer %** |
|---|---|---|
| Phenylalanin/LS90P/Protein | 80/10/10 | 98 |
| Phenylalanin/LS90P/Protein | 80/15/5 | 98 |
| Phenylalanin/LS90P/Protein | 60/30/10 | 99 |
| Phenylalanin/LS90P/Protein | 70/25/5 | 98 |

### BEISPIEL 3 LAGERSTABILITÄT

In den vorhergehenden Beispielen konnte gezeigt werden, dass Phenylalanin alleine und im besonderen in Kombination mit einem Zucker sehr gute aerodynamische Eigenschaften von Pulver nach Sprühtrocknung hervorbringt. Phenylalanin allein vermag jedoch nicht jedes Protein, z.B. den in den Beispielen 1 und 2 verwendeten IgG1-Antikörper, zu stabilisieren. Für solche Proteine ist jedoch eine Stabilisierung durch Zugabe von Zucker möglich.

In diesem Beispiel wurde nun die Lagerstabilität nach Sprühtrocknung untersucht. Hierbei wurde zum einen der Phenylalanin-Anteil variiert (80-60% bezogen auf das Pulver). Zum anderen wurde der Einfluss des LS90P-Anteils auf die Proteinstabilität untersucht. Es wurden verschiedene Verhältnisse zwischen Protein und Zucker eingesetzt (siehe Tabelle 5 und 6).

**Tabelle 5: Zusammensetzung Sprühlösung**

| | Lösung 1 | Lösung 2 | Lösung 3 |
|---|---|---|---|
| Phenylalanin: | 2,29 g / 100 mL | 3,06 g / 100 mL | 2,29 g / 100 mL |
| IgG1: | 1,15 g / 100 mL | 338 g / 100 mL | 383 mg / 100 mL |
| LS90P: | 383 mg / 100 mL | 383 mg / 100 mL | 1,15 g / 100 mL |
| Feststoffanteil: | 3,82 % | 3,82 % | 3,82 % |
| Protein/Zucker Massenverhältnis | 3:1 | 1:1 | 1:3 |

Das Phenylalanin wurde unter Erhitzen (80°C) in Lösung gebracht. Nach Abkühlen der Lösung auf Raumtemperatur wurde das Protein und der Zucker zugegeben.

**Tabelle 6: Zusammensetzung sprühgetrocknete Pulver**

| | Pulver 1 | Pulver 2 | Pulver 3 |
|---|---|---|---|
| Phenylalanin: | 60 % | 80 % | 60 % |
| IgG1: | 30 % | 10 % | 10 % |
| LS90P: | 10 % | 10 % | 30 % |
| Protein/Zucker Verhältnis | 3:1 | 1:1 | 1:3 |

Die Lösungen wurden unter folgenden Sprühbedingungen sprühgetrocknet:
- Sprühtrockner:: SD-Micro (Fa. Niro)
- Engangstemperatur: 150 °C
- Ausgangstemperatur:: 90 °C
- Zerstäubergasrate:: 4 kg/h
- Trocknungsgasrate:: 28 kg/h

Lagerbedingungen: Die Pulver wurden für 3 Monate bei verschiedenen Lagerbedingungen (25 °C / trocken, 40 °C / trocken, 25 °C / 60 %rF) eingelagert (siehe Tabelle 7 und 8). Für die Lagerbedingung 25 °C / trocken und 40 °C /trocken wurde das Pulver unter trockenen Bedingungen (<30 %rF) in Glasflaschen abgefüllt und mit Gummistopfen und Bördelkappe verschlossen. Die Lagerung bei 25°C und 60% relative Feuchte erfolgte über eine gesättigte Salzlösung im Exikkator. Der Exikkator wurde im Trockenschrank temperiert.

**Tabelle 7: MMAD in µm**

| | Anfangswert | 2 Wochen | 1 Monat | | | 2 Monate | | 3 Monate | | |
|---|---|---|---|---|---|---|---|---|---|---|
| | | 40°C, trocken | 25°C, trocken | 25°C, 60% | 40°C, trocken | 25°C, trocken | 25°C/ 60% | 25°C, trocken | 25°C/ 60% | 40°C, trocken |
| Pulver 1 | 4,25 | 4,21 | 4,25 | 4,16 | 4,45 | 4,11 | 4,21 | 4,0 | 4,4 | 4,6 |
| Pulver 2 | 3,77 | 3,51 | 3,39 | 3,73 | 3,57 | 3,56 | 3,46 | 3,5 | 3,8 | 3,7 |
| Pulver 3 | 3,73 | 3,75 | 3,80 | 3,68 | 3,81 | 3,75 | 4,03 | 3,8 | 3,9 | 4,0 |

Der MMAD zeigt keine signifikanten Abhängigkeiten zwischen den Chargen und den Lagerbedingungen.

**Tabelle 8: FPF in Prozent**

| | Anfangswert | 2 Wochen | 1 Monat | | | 2 Monate | | 3 Monate | | |
|---|---|---|---|---|---|---|---|---|---|---|
| | | 40°C, trocken | 25°C, trocken | 25°C, 60% | 40°C, trocken | 25°C, trocken | 25°C/ 60% | 25°C, trocken | 25°C/ 60% | 40°C, trocken |
| Pulver 1 | 59,6 | 53,6 | 59,6 | 60,5 | 55,9 | 46,1 | 66,6 | 50,9 | 53,4 | 40,1 |
| Pulver 2 | 51,2 | 54,0 | 54,8 | 64,5 | 53,6 | 59,2 | 66,7 | 45,2 | 59,3 | 39,0 |
| Pulver 3 | 45,6 | 49,9 | 47,7 | 58,3 | 55,5 | 40,6 | 55,1 | 40,7 | 55,1 | 36,6 |

Die FPF liegt direkt nach Herstellung, also vor Einlagerung bei 46 % (Pulver 3) bis 60 % (Pulver 1). Eine Erniedrigung des Phenylalanin-Gehaltes von 80 % (Pulver 2) auf 60 % (Pulver 3) wirkt sich nicht negativ auf die Feinpartikelfraktion aus.

**Tabelle 9: Monomergehalte des IgG1 Antikörpers im sprühgetrocknetem Pulver**

| | Anfangswert | 2 Wochen | 1 Monat | | | 2 Monate | | 3 Monate | | |
|---|---|---|---|---|---|---|---|---|---|---|
| | | 40°C, trocken | 25°C, trocken | 25°C, 60% | 40°C, trocken | 25°C, trocken | 25°C/ 60% | 25°C, trocken | 25°C/ 60% | 40°C, trocke n |
| Pulver 1 | 97,0 | 96,0 | 97,3 | 90,7 | 97,2 | 97,6 | 90,4 | 97,4 | 88,0 | 95,4 |
| Pulver 2 | 92,0 | 90,6 | 92,1 | 80,9 | 93,0 | 93,1 | 80,9 | 93,1 | 78,3 | 90,9 |
| Pulver 3 | 96,3 | 96,1 | 94,9 | 93,1 | 96,3 | 97,2 | 93,0 | 96,1 | 92,0 | 96,1 |

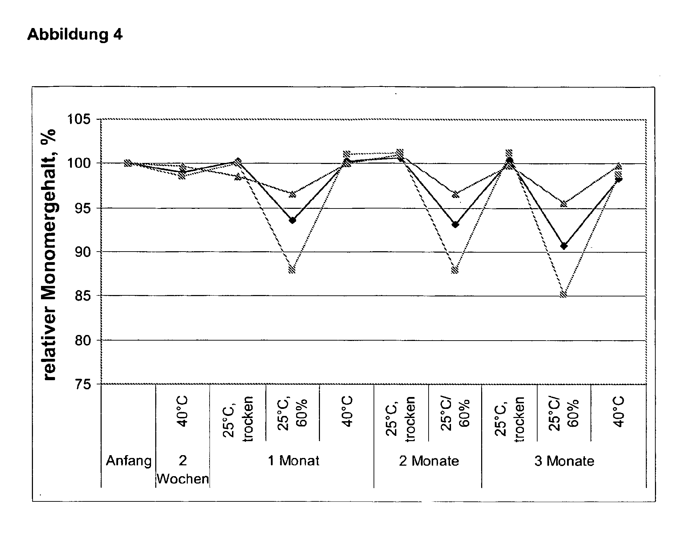

Die Proteinstabilität nach Sprühtrocknung und Einlagerung wird in Tabelle 9 dargestellt. Die Tabelle 9 zeigt die prozentualen Monomergehalte des IgG1 Antikörpers. In Abbildung 4 werden die relativen Monomergehalte bezogen auf den Anfangswerte dargestellt.

Das Beispiel verdeutlicht, dass das Protein unter trockenen Lagerbedingungen sowohl bei 25 °C als auch bei 40 °C über die geprüfte Lagerzeit annähernd vollständig stabilisiert gelagert werden kann. Unter feuchten Bedingungen kommt es zu einer leichten Schädigung des im Beispiel verwendeten Antikörpers.

Die ternären Pulver haben also eine gute Feinpartikelfraktion und zusätzlich auch eine gute Lagerstabilität.

### BEISPIEL 4 LAGERSTABILITÄT IM VERGLEICH (DEXTRAN- UND PHENYLALANIN-HALTIGE PULVER)

Die Eigenschaften Phenylalanin-haltiger Pulver wurden mit den Eigenschaften anderer, konventioneller Pulver verglichen (siehe Tabelle 10). Bei beiden Pulvern kommt es über die Lagerung nur zu einer geringfügigen Änderung der aerodynamischen Teilchengröße (Tabelle 11).

**Tabelle 10: Die Sprühlösungen wurden unter folgenden Sprühbedingungen sprühgetrocknet:**

| | **Phenylalanin/LS90P/IgG 1 (60/10/30)** | **Dextran 1/Isoleucin/IgG1 (65/5/30)** |
|---|---|---|
| Sprühtrockner | SDMicro | SDMicro |
| Feststoffanteil | 7,5 % | 3,8% |
| Eingangstemperatur | 150 °C | 150 °C |
| Ausgangstemperatur | 85 °C | 90 °C |
| Zerstäubergasrate | 5 kg/h | 4 kg/h |
| Trocknungsgasrate | 28 kg/h | 28 kg/h |

**Tabelle 11 MMAD [µm]**

| **Lagerzeit** | **Lagerbedingung** | **Dextran/Isoleucin/IgG1 (65/5/30)** | **Phenylalanin/LS90P/ IgG1 (60/10/30)** |
|---|---|---|---|
| **Anfang** | **--** | 3,81 | 4,25 |
| **2 Wochen** | **40 °C, trocken** | 3,58 | 4,21 |
| **1 Monat** | **25 °C, trocken** | 3,80 | 4,25 |
| | **25 °C / 60 %** | 4,23 | 4,16 |
| | **40 °C, trocken** | 3,63 | 4,45 |
| **2 Monate** | **25 °C, trocken** | 4,00 | 4,11 |
| | **25 °C / 60 %** | 4,40 | 4,21 |
| **3 Monate** | **25 °C, trocken** | 3,67 | 4,00 |
| | **25 °C / 60 %** | 4,47 | 4,40 |

**Tabelle 12: Feinpartikelfraktionen**

| **Lagerzeit** | **Lagerbedingung** | **Dextran/Ilsoleucin/IgG1 (65/5/30)** | | **Phenylalanin/LS90P/IgG1 (60/10/30)** | |
|---|---|---|---|---|---|
| | | **FPF, %** | **relative FPF bezogen auf den Anfangswert, %** | **FPF, %** | **relative FPF bezogen auf den Anfangswert, %** |
| **Anfang** | | 33,7 | 100,0 | 59,6 | 100,0 |
| **2 Wochen** | **40 °C, trocken** | 21,1 | 62,7 | 53,6 | 89,9 |
| **1 Monat** | **25 °C, trocken** | 26,2 | 77,9 | 59,6 | 100,0 |
| | **25 °C / 60 %** | 16,4 | 48,8 | 60,5 | 101,5 |
| | **40 °C, trocken** | 22,4 | 66,5 | 55,9 | 93,8 |
| **2 Monate** | **25 °C, trocken** | 24,0 | 71,1 | 46,1 | 77,3 |
| | **25 °C / 60 %** | 14,6 | 43,4 | 66,6 | 111,7 |
| **3 Monate** | **25 °C, trocken** | 23,3 | 69,1 | 50,9 | 85,4 |
| | **25 °C / 60%** | 15,2 | 45,1 | 53,4 | 89,6 |

Das Phenylalanin-haltige Pulver hat verglichen zum Dextran-haltigen Pulver eine wesentlich bessere FPF (59,6 % vers. 33,7 %, siehe Tabelle 12 / Abbildung 5). Da die aerodynamischen Teilchengrößen der beiden Pulver nur geringfügig unterschiedlich sind bzw. das Phenylalanin-haltige Pulver sogar einen leicht erhöhten MMAD aufweist (siehe Tabelle 11), sind die Unterschiede der FPF auf das Dispergierverhalten der Pulver beim Ausbringen aus der Kapsel zurück zu führen. Das bedeutet, dass sich das Phenylalanin-haltige Pulver wesentlich besser dispergieren lässt und somit interpartikuläre Wechselwirkungen geringer sind, verglichen zum entsprechenden Dextran-haltigen Pulver.

In Abbildung 6 ist die relative FPF bezogen auf den Anfangswert der Stabilität dargestellt. Es zeigt sich hier, dass das Phenylalanin-haltige Pulver über die Lagerzeit wesentlich geringere Einbrüche in der FPF aufweist. Besonders vorteilhaft ist das Phenylalanin bei höheren Luftfeuchten. Während beim Dextran-haltigen Pulver die FPF auf 45-49 % des Ausgangswertes abfällt, zeigt das Phenylalanin-haltige Pulver nach 2 Monaten Lagerung sogar einen Anstieg in der FPF und nach 3 Monaten nur einen leichten Abfall auf 89 % des Ausgangswertes.

Dieses Ergebnis unterstreicht im Besonderen die Eignung der ternären Pulverzusammensetzungen bei erhöhten Luftfeuchten. Die herkömmlichen sprühgetrockneten Pulver zeigen in der Regel einen starken Einbruch im aerodynamischen Verhalten. Durch das Phenylalanin kommt es dagegen zu einer Stabilisierung der Aerodynamik bzw., wie in diesem Beispiel gezeigt, zu einer Verbesserung derselben.

### Morphologie der Pulver:

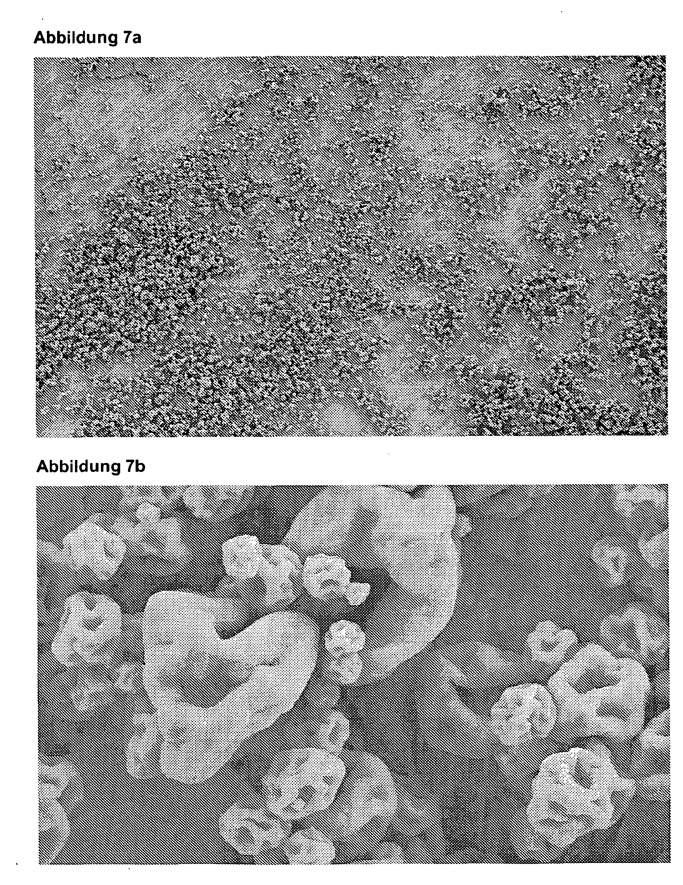

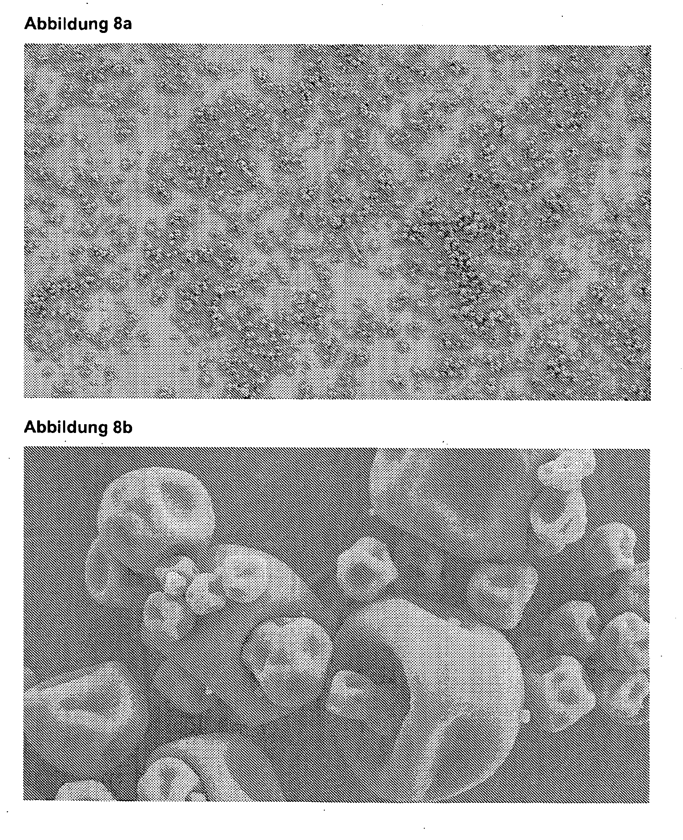

Wie in Abbildung 7 und Abbildung 8 zu erkennen, zeigen beide Pulver (Phenylalanin-haltiges Pulver Abbildung 7, Dextran-haltiges Pulver Abbildung 8) keine größeren Pulveragglomerate. Des weiteren sind bei beiden Formulierungen Mehrfachdellen zu sehen. Ein wesentlicher Unterschied in den beiden Morphologien ist die höhere Oberflächenrauigkeit beim Phenylalanin-haltigen Pulver. Diese erhöhte Oberflächenrauigkeit ist vermutlich auch der Grund für ein besseres Dispergierverhalten.

Durch die Zugabe der hydrophoben Aminosäuren (Isoleucin bzw. Phenylalanin) sollte bei beiden Pulvern die Oberflächen der Partikel zumindest zum Teil hydrophob sein. Hieraus ergibt sich wiederum, dass das alleinige Hydrophobisieren der Oberfläche hinsichtlich der aerodynamischen Eigenschaften weit weniger effizient ist, als das Induzieren rauher Oberflächenstrukturen, wie es beim Phenylalanin der Fall ist.

### BEISPIEL 5 SPRÜHTROCKNUNG BEI UNTERSCHIEDLICHEN PH- WERTEN

In diesem Beispiel wurden verschiedene pH-Werte der Sprühlösung einer definierten Zusammensetzung (siehe Tabelle 13) eingestellt und versprüht.

Die Sprühbedingungen sind in Tabelle 14 dargestellt.

**Tabelle 13: Zusammensetzung der Sprühlösungen**

| | Sprühlösung 1 | Sprühlösung 2 | Sprühlösung 3 |
|---|---|---|---|
| Phenylalanin: | 2,29g/100mL | 2,29g/100mL | 2,29g/100mL |
| IgG1: | 1,15g/100mL | 1,15g/100mL | 1,15g/100mL |
| LS90P: | 383mg/100mL | 383mg/100mL | 383mg/100mL |
| Puffer | 1,6mMGlycin / 25mM Histidin, pH 5,9 | 25mM TRIS, pH 7,4 | 25mM TRIS pH 9,0 |
| Feststoffanteil | 4,2% (w/w) | 4,1 % (w/w) | 4,1 % (w/w) |

**Tabelle 14: Sprühbedingungen**

| Sprühtrockner | Büchi B191 |
|---|---|
| Eingangstemperatur | 150 °C |
| Ausgangstemperatur | 90 °C |
| Zerstäubergasrate | 700 L/h |
| Trocknungsgasrate | 100% Aspiratorleistung |

**Tabelle 15: Ergebnisse**

| | Pulver 1 | Pulver 2 | Pulver 3 |
|---|---|---|---|
| MMAD, µm | 3,9 | 4,4 | 5,2 |
| FPF, % | 44,7 | 51,8 | 48,9 |
| ausgebrachte Masse, % | 91,7 | 87,0 | 89,2 |
| Kristallinität | Teilkristallin | Teilkristallin | Teilkristallin |
| Monomergehalt, % | 98 | 97 | 90 |

Das aerodynamische Verhalten (FPF, ausgebrachte Masse) der in Tabelle 15 dargestellten Pulver zeigen keine wesentlichen Unterschiede. Die hergestellten Pulver waren jeweils teilkristallin. Demnach ist der pH-Wert der Sprühlösung nicht entscheidend für die Pulvereigenschaften (Dispergierbarkeit / Inhalierbarkeit) und der Sprüheigenschaft des Phenylalanins.

Die Proteinstabilisierung hängt vom pH-Wert der Sprühlösung ab. Der eingesetzte Antikörper ist bei niedrigen pH-Werten stabiler. Dennoch kann auch bei hohen pH-Werten von 9,0 eine Proteinstabilisierung im besonderen im Vergleich zu binären Zusammensetzungen (siehe Abbildung 2) erreicht werden.

### BEISPIEL 6 SPRÜHTROCKNUNGEN MIT VERSCHIEDENEN PHENYLALANIN-ANTEILEN

In diesem Beispiel wird der Phenylalanin-Anteil im sprühgetrockneten Pulver von 50% w/w auf 20% w/w reduziert. Die Zusammensetzungen im Pulver sind in Tabelle 16 dargestellt. Die Sprühbedingungen sind in Tabelle 17 dargestellt.

**Tabelle 16: Zusammensetzung der sprühgetrockneten Pulver**

| | Pulver 1 | Pulver 2 | Pulver 3 | Pulver 4 |
|---|---|---|---|---|
| Phenylalanin | 50%w/w | 40%w/w | 30%w/w | 20%w/w |
| IgG1 | 30%w/w | 30%w/w | 30%w/w | 30%w/w |
| LS90P | 20%w/w | 30%w/w | 40%w/w | 50%w/w |

**Tabelle 17: Sprühbedingungen**

| Sprühtrockner | Büchi B191 |
|---|---|
| Feststoffanteil | 3,82 % |
| Eingangstemperatur | 150 °C |
| Ausgangstemperatur | 90 °C |
| Zerstäubergasrate | 700 L/h |
| Trocknungsgasrate | 100% Aspiratorleistung |

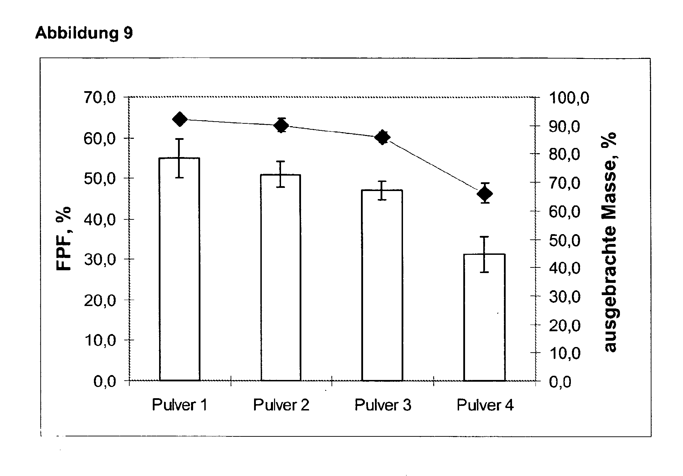

Die Abbildung 9 zeigt das aerodynamische Verhalten der sprühgetrockneten Pulver in Abhängigkeit zum Phenylalanin-Anteil im Pulver. Dieser Abbildung folgend kann der Phenylalanin-Anteil im sprühgetrockneten Pulver auf 30% (w/w) reduziert werden. Bei einer weiteren Reduzierung des Phenylalanin-Anteils auf 20% (w/w) verringert sich sowohl die Feinpartikelfraktion als auch die ausgebrachte Masse erheblich.

Die Partikelmorphologie hängt stark vom Phenylalanin-Anteil im sprühgetrockneten Pulver ab. Bei 50% (w/w), 40% (w/w) und 30% (w/w) Phenylalanin-Anteil liegen stark gefaltete, rosinen-artige Partikel vor (Abbildung 10a - 10c). Bei einer Reduzierung des Phenylalanin-Anteils auf 20% nimmt die Faltungsintensität stark ab. Die Änderung der Partikelmorphologie korreliert mit der Verschlechterung des aerodynamischen Verhaltens des Pulvers. Das bedeutet, das der positive Effekt des Phenylalanins beim Sprühtrocknen von Sprühlösungen erst ab 30% (w/w) deutlich wird.

### BEISPIEL 7 SPRÜHTROCKNUNG VERSCHIEDENER PROTEINE

In diesem Beispiel wurden neben einem Antikörper vom Typ IgG das Hormon Calcitonin und das Enzym Lysozym sprühgetrocknet. Die Zusammensetzungen der hergestellten Pulver sind in Tabelle 18 und die Sprühbedingungen in Tabelle 19 dargestellt.

**Tabelle 18: Zusammensetzung der sprühgetrockneten Pulver**

| Pulver 1 | Pulver 2 | Pulver 3 |
|---|---|---|
| 60%w/w Phenylalanin | 60%w/w Phenylalanin | 60%w/w Phenylalanin |
| 10%w/w IgG | 10%w/w Lysozym | 10%w/w Calcitonin |
| 30%w/w LS90P | 30%w/w LS90P | 30%w/w LS90P |

**Tabelle 19: Sprühbedingungen**

| Sprühtrockner | Büchi B191 |
|---|---|
| Feststoffanteil | 3,8 %w/v |
| Eingangstemperatur | 150 °C |
| Ausgangstemperatur | 90 °C |
| Zerstäubergasrate | 700 L/h |
| Trocknungsgasrate | 100% Aspiratorleistung |

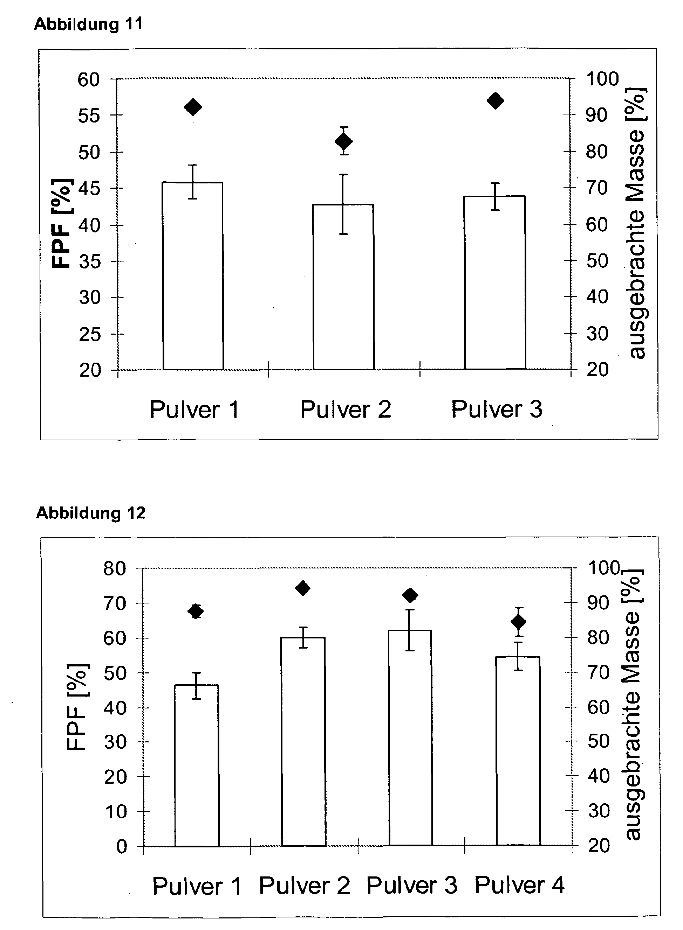

In Abbildung 11 ist die Feinpartikelfraktion sowie die ausgebrachte Masse der sprühgetrockneten Pulver 1-3 dargestellt. Die Art des Proteins ist demnach nicht maßgeblich für das aerodynamische Verhalten der sprühgetrockneten Pulver.

### BEISPIEL 8 HERSTELLUNG SPRÜHGETROCKNETER PULVER MIT VERSCHIEDENEN WEITEREN HILFSSTOFFEN

In dieser Versuchsreihe wurden im Austausch zum LS90P weitere Hilfsstoffe mit Phenylalanin und einem IgG1 Antikörper sprühgetrocknet. Die Zusammensetzungen der hergestellten Pulver sind in Tabelle 20 dargestellt, die Sprühbedingungen in Tabelle 21.

**Tabelle 20: Zusammensetzung der sprühgetrockneten Pulver**

| Pulver 1 | Pulver 2 | Pulver 3 | Pulver 4 |
|---|---|---|---|
| 60%w/w Phenylalanin | 60%w/w Phenylalanin | 60%w/w Phenylalanin | 60%w/w Phenylalanin |
| 30%w/w IgG | 30%w/w IgG | 30%w/w IgG | 30%w/w IgG |
| 10 %w/w Saccharose | 10%w/w Mannitol | 10%w/w Glycin | 10%w/w Polyvinylpyrrolividon (PVP) |

**Tabelle 21: Sprühbedingungen**

| Sprühtrockner | Büchi B191 |
|---|---|
| Feststoffanteil | 3,8 % |
| Eingangstemperatur | 150 °C |
| Ausgangstemperatur | 90 °C |
| Zerstäubergasrate | 700 L/h |
| Trocknungsgasrate | 100% Aspiratorleistung |

In Abbildung 12 sind die Feinpartikelfraktionen und die ausgebrachten Massen dargestellt. Die Feinpartikelfraktionen sind bei den getesteten Hilfsstoffen sehr hoch (Saccharose: 46%, Mannitol: 60%, Glycin: 62%, PVP: 63%). Durch eine geschickte Wahl der Hilfsstoffe kann der positive Effekt des Phenylalanins auf den Sprühtrocknungsprozess weiter verbessert werden. Hierbei ist der weitere Hilfsstoff nicht auf eine Stoffklasse beschränkt. Es kann sich, wie in diesem Beispiel gezeigt, um einen Zucker oder Zuckeralkohol, eine Aminosäure oder auch um ein Polymer handeln. Entscheidend für die Verwendung des weiteren Hilfsstoffes ist die Stabilisierung des Proteins bei der Sprühtrocknung. In Tabelle 22 sind die Monomergehalte des eingesetzten Antikörpers dargestellt. Es zeigt sich, dass durch die Zugabe eines weiteren Hilfsstoffes das Protein verglichen zu binären Gemischen aus Phenylalanin und IgG1 (siehe Abbildung 2) stabilisiert werden kann.

**Tabelle 22**

| | Pulver 1 | Pulver 2 | Pulver 3 | Pulver 4 |
|---|---|---|---|---|
| Monomergehalt IgG1 | 98 % | 98 % | 95 % | 94 % |

### BEISPIEL 9 SPRÜHTROCKNUNG UNTER EINSATZ VON KRISTALLISATIONSINHIBITOREN

In diesem Beispiel soll gezeigt werden, dass durch den Einsatz von Kristallisationsinhibitoren die sprühgetrockneten Pulver optimiert werden können. Zu diesem Zweck wurden entsprechend Tabelle 23 verschiedene Pulver hergestellt.

**Tabelle 23 Zusammensetzungen der Pulver**

| | Zusammensetzung | Herstellverfahren |
|---|---|---|
| Pulver 1 | 60% Phenylalanin | Sprühtrocknung |
| | 40% LS90P | (SDMicro) |
| Pulver 2 | 60% Phenylalanin | Sprühtrocknung |
| | 30% LS90P | (SDMicro) |
| | 10% IgG1 | |
| Pulver 3 | 60% Phenylalanin | Sprühtrocknung |
| | 30% LS90P | (Büchi B191) |
| | 10% Lysozym | |
| Pulver 4 | 60% Phenylalanin | Sprühtrocknung |
| | 30% LS90P | (Büchi B191) |
| | 10% Calcitonin | |
| Pulver 5 | 100% LS90P | Gefriertrocknung |
| | | (GT-12B) |

Die Sprühbedingungen am Büchi B191 und am SDMicro sind in Tabelle 24 zusammengefaßt.

**Tabelle 24 Sprühbedingungen**

| Sprühtrockner | Büchi B191 | SDMicro |
|---|---|---|
| Feststoffanteil | 3,8 % | 3,8 % |
| Eingangstemperatur | 150 °C | 150 °C |
| Ausgangstemperatur | 90 °C | 90 °C |
| Zerstäubergasrate | 700 L/h | 4 kg/h |
| Trocknungsgasrate | 100% Aspiratorleistung | 28 kg/h |

Ziel der Gefriertrocknung einer wässrigen LS90P-Lösung war die Herstellung von röntgenamorphem Pulver. Hierzu wurde eine wässrige Lösung mit geringem Feststoffanteil (5g/100mL) hergestellt und entsprechend wie in Tabelle 25 beschrieben gefriergetrocknet.

**Tabelle 25 Temperatur- und Druckprogramm der Gefriertrocknung**

| Prozessschritt | Zeitt | Temperatur | Druck [mbar] |
|---|---|---|---|
| | [hh:mm] | [°C] | |
| Start | - | 20 | - |
| Gefrieren | 01:30 | -50 | - |
| (Temperatur-Rampe) | | | |
| Gefrieren (Halteschritt) | 06:30 | -50 | - |
| Nachtrocknung | 00:01 | -50 | 0,016 |
| (Druck-Rampe) | | | |
| Hauptrocknung | 07:00 | -40 | 0,016 |
| (Temperatur-Rampe) | | | |
| Hauptrocknung | 23:00 | -40 | 0,016 |
| (Halteschritt) | | | |
| Hauptrocknung | 03:20 | -23 | 0,016 |
| (Temperatur-Rampe) | | | |
| Hauptrocknung | 30:00 | -23 | 0,016 |
| (Halteschritt) | | | |
| Hauptrocknung | 02:00 | 20 | 0,016 |
| (Temperatur-Rampe) | | | |
| Nachtrocknung | 00:01 | 20 | 0,001 |
| (Druck-Rampe) | | | |
| Nachtrocknung | 17:00 | 20 | 0,001 |
| (Halteschritt) | | | |

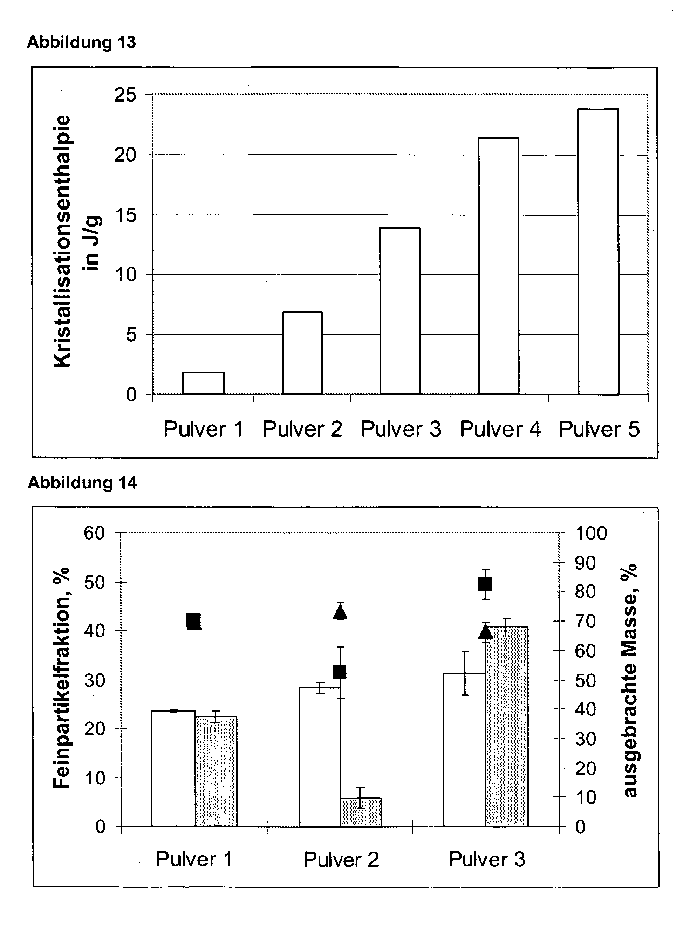

In Abbildung 13 sind die Rekristallisationsenthalpien vom LS90P nach dem Erhitzen der Pulver in einem DSC-Gerät (DSC821/Mettler Toledo) dargestellt. Es zeigt sich, dass die Kristallisationsenthalpie bezogen auf den Massenanteil des eingesetzten Proteins stark vom Protein abhängt. So nimmt die Kristallisationsenthalpie in der Reihe IgG1 (6,8J/g), Lysozym (13,9J/g), Calcitonin (21,3J/g) und damit auch der amorphe Anteil des LS90P nach Sprühtrocknung zu.

Da das LS90P in den jeweiligen Pulverformulierungen die Proteinstabilisierende Komponente im Pulver ist, ist ein hoher amorpher Anteil des LS90P im Pulver erwünscht. In einer weiteren Versuchsreihe wurde daher als Kristallisationsinhibitor HSA der Sprühlösung zugesetzt. Die Sprühtrocknung erfolgte analog Tabelle 26. Die Zusammensetzung des Pulvers war:
60% Phenylalanin / 30% LS90P / 1% HSA / 9% IgG1.

Die Kristallisationsenthalpie vom LS90P lag bei 24,3J/g und entspricht dem röntgenamorphen LS90P (23,8J/g). Bezogen auf das IgG1-haltige Pulver 2 kann durch die Zugabe geringer Mengen an HSA die Pulvereigenschaften bezogen auf den amorphen Charakter des Pulvers optimiert werden.

**Tabelle 26 Sprühbedingungen**

| Sprühtrockner | SDMicro |
|---|---|
| Feststoffanteil | 3,8 % |
| Eingangstemperatur | 150 °C |
| Ausgangstemperatur | 100 °C |
| Zerstäubergasrate | 4 kg/h |
| Trocknungsgasrate | 28 kg/h |

### BEISPIEL 10 VERGLEICH VERSCHIEDENER AROMATISCHER AMINOSÄUREN

In diesem Beispiel sollen die aromatischen Aminosäuren Tryptophan und Histidin vergleichbaren Phenylalanin-haltigen Pulvern gegenüber gestellt werden. Die aromatische Aminosäure Tyrosin scheidet als potentieller Hilfsstoff für die Sprühtrocknung aus, da diese Aminosäure zu schlecht wasserlöslich ist. Tryptophan ist verglichen zum Phenylalanin ebenfalls sehr schlecht wasserlöslich, so dass zur Herstellung pharmazeutisch relevanter Pulver maximale Tryptophan-Anteile von 20%w/w realisierbar sind.

Um die Sprüheigenschaften der aromatischen Aminosäuren zu vergleichen, wurden jeweils Pulver mit 20% Aminosäureanteil hergestellt. In Tabelle 27 sind die Zusammensetzungen der Pulver und in Tabelle 28 die Sprühbedingungen zusammengefasst.

**Tabelle 27: Zusammensetzung der sprühgetrockneten Pulver**

| Pulver 1 | Pulver 2 | Pulver 3 |
|---|---|---|
| 20%w/w Tryptophan | 20%w/w Histidin | 20%w/w Phenylalanin |
| 30%w/w IgG 1 | 30%w/w IgG 1 | 30%w/w IgG 1 |
| 50%w/w LS90P | 50%w/w LS90P | 50%w/w LS90P |

**Tabelle 28: Sprühbedingungen**

| Sprühtrockner | Büchi B191 |
|---|---|
| Feststoffanteil | 3,8 % |
| Eingangstemperatur | 150 °C |
| Ausgangstemperatur | 90 °C |
| Zerstäubergasrate | 700 L/h |
| Trocknungsgasrate | 100% Aspiratorleistung |

Die Feinpartikelfraktionen waren nach Sprühtrocknung beim Phenylalanin-haltigen Pulver geringfügig besser (siehe Abbildung 14).

Ein wesentlicher Vorteil des Phenylalanin-haltigen Pulver gegenüber dem Histidin-haltigen Pulver liegt in der geringeren Feuchteempfindlichkeit. Während die FPF des Histidin-haltigen Pulvers nach einer Exposition mit 50% relativer Luftfeuchte von ursprünglich 28% auf 6% einbricht, kommt es beim Phenylalanin-haltigen Pulver nach Feuchteexposition sogar zu einer Optimierung der FPF. Entsprechendes Verhalten ist auch für die ausgebrachte Masse zu erkennen. Beim Histidin-haltigen Pulver nimmt die ausgebrachte Masse ab, beim Phenylalain-haltigen Pulver dagegen zu.

Das Tryptophan-haltige Pulver zeigt keine Änderung in der FPF und ausgebrachten Masse durch Luftfeuchte. Nachteilig an dieser Aminosäure gegenüber dem Phenylalanin ist, wie bereits oben erwähnt, die sehr geringe Wasserlöslichkeit.

Histidin wurde weiterführend mit entsprechenden Phenylalanin-haltigen Pulvern verglichen (siehe Tabelle 29). Die Herstellungen erfolgten analog den in Tabelle 28 beschriebenen Sprühbedingungen.

**Tabelle 29**

| Pulver 4 | Pulver 5 | Pulver 6 | Pulver 7 |
|---|---|---|---|
| 30%w/w Histidin | 60%w/w Histidin | 30%w/w Phenylalanin | 60%w/w Phenylalanin |
| 30%w/w IgG 1 | 30%w/w IgG 1 | 30%w/w IgG 1 | 30%w/w IgG 1 |
| 50%w/w LS90P | 10%w/w LS90P | 50%w/w LS90P | 10%w/w LS90P |

Während die Pulver 4 und 6 ähnliche aerodynamische Eigenschaften besitzen, zeigt das Phenylalanin-haltige Pulver 7 verglichen zum entsprechenden Histidin-haltigen Pulver 5 eine wesentlich bessere Feinpartikelfraktion (siehe Tabelle 30). Besonders deutlich ist der Unterschied der Aerodynamik nach einer Feuchteexposition (siehe Tabelle 31). Durch den Einfluss von Feuchtigkeit bricht die FPF bei den getesteten Histidin-haltigen Pulver annähernd komplett ein. Phenylalanin-haltige Pulver zeugen dagegen eine leichte Verbesserung im aerodynamischen Verhalten.

**Tabelle 30: FPF und ausgebrachte Masse sprühgetrockneter Pulver ohne Feuchtekonditionierung**

| Pulver | FPF, % | Ausgebrachte Masse, µm |
|---|---|---|
| Pulver 4 | 40,5 | 88,5 |
| Pulver 5 | 28,1 | 84,4 |
| Pulver 6 | 47,0 | 85,9 |
| Pulver 7 | 49,7 | 92,1 |

**Tabelle 31: FPF und ausgebrachte Masse sprühgetrockneter Pulver mit Feuchtekonditionierung (50% Feuchte / 20 Stunden / Raumtemperatur)**

| Pulver | FPF, % | Ausgebrachte Masse, µm |
|---|---|---|
| Pulver 4 | 3,3 | 66,6 |
| Pulver 5 | 5,4 | 70,5 |
| Pulver 6 | 57,2 | 85,9 |
| Pulver 7 | 54,9 | 88,6 |

Zusammenfassend kann festgestellt werden, dass die positiven Eigenschaften des Phenylalanins auf die Sprühtrocknung nicht durch andere aromatische Aminosäuren erzielbar sind.

## Patentansprüche

1. Pulver enthaltend ein Protein und Phenylalanin, **dadurch gekennzeichnet, dass** das Pulver mindestens 30 % (w/w) Phenylalanin, bevorzugt mindestens 40 % (w/w) Phenylalanin enthält.

2. Pulver gemäß Anspruch 1, **dadurch gekennzeichnet, dass** das Pulver mindestens einen weiteren Hilfsstoff wie einen Zucker oder ein Polyol enthält.

3. Pulver gemäß Anspruch 1 oder 2, **dadurch gekennzeichnet, dass** das Pulver sprühgetrocknet ist.

4. Pulver gemäß einem der Ansprüche 2 bis 3 **dadurch gekennzeichnet, dass** der Zucker ein nicht reduzierender Zucker ist ausgewählt aus der Gruppe bestehend aus einem Disaccharid und einem Oligosaccharid.

5. Pulver gemäß einem der Ansprüche 1 bis 3 **dadurch gekennzeichnet, dass** das Protein ein Wirkstoff ist, bevorzugt ein pharmazeutischer Wirkstoff wie ein Antikörper, ein Antikörperfragment, ein Fusionsprotein mit Teilen von Antikörpern oder ein konjugierter Antikörper, ein Wachstumsfaktor, ein Hormon oder ein Enzym.

6. Pulver gemäß einem der Ansprüche 1 bis 5 **dadurch gekennzeichnet, dass** die mittlere aerodynamische Teilchengröße (MMAD = Mass median aerodynamic diameter) der Pulverpartikel kleiner 10 µm, bevorzugt kleiner 7,5 µm, noch bevorzugt im Bereich zwischen 1-6 µm bzw. 3-6 µm oder 5-7 µm liegt.

7. Pharmazeutische Zusammensetzung enthaltend ein Pulver gemäß einer der Ansprüche 1 bis 6.

8. Verfahren zur Herstellung eines Pulvers gemäß einem der Ansprüche 1 bis 6 **dadurch gekennzeichnet, dass**
a) eine Phenylalaninlösung hergestellt wird,
b) mindestens ein Protein und optional mindestens ein weiterer Hilfsstoff wie ein Zucker oder ein Polyol hinzugefügt werden,
c) die so erhaltene Lösung oder Suspension bei einer Einströmtemperatur von vorzugsweise 90 - 200 °C und einer Ausströmtemperatur von vorzugsweise 40 - 150 °C versprüht wird und
d) die entstandenen Partikel vom Trocknungsgas abgetrennt werden.

9. Verfahren gemäß Anspruch 8 **dadurch gekennzeichnet, dass** es sich bei dem Protein um einen pharmazeutischen Wirkstoff handelt.

10. Verfahren gemäß Anspruch 8 oder 9 **dadurch gekennzeichnet, dass** zwischen dem Schritt a) und b) folgende weitere Schritte durchgeführt werden
- eine Erwärmung der Phenylalaninlösung, vorzugsweise auf 80°C,
- eine Abkühlung der Phenylalaninlösung bis unterhalb der Denaturierungstemperatur des jeweils hinzuzufügenden Proteins, wobei die Abkühlung bevorzugt bis auf Raumtememperatur erfolgt.

11. Verfahren gemäß Anspruch 8 bis 10 **dadurch gekennzeichnet, dass** die Lösung oder Suspension im Schritt c) mittels mindestens einer Druckdüse oder mindestens eines Rotationszerstäubers oder mindestens einer Venturidüse oder mindestens eines Ultraschallverneblers oder mindestens einer Zweistoffdüse versprüht wird.

12. Verfahren gemäß Anspruch 8 bis 11 **dadurch gekennzeichnet, dass** die Abtrennung der Partikel in Schritt d) mittels mindestens eines Partikelabscheiders erfolgt, bevorzugt mittels mindestens eines Zyklons.

13. Pulver gemäß der Ansprüche 1 bis 6 oder pharmazeutische Zusammensetzung gemäß Anspruch 7 zur Verwendung als Arzneimittel.

14. Pulver gemäß einem der Ansprüche 1 bis 6 oder eine pharmazeutische Zusammensetzung gemäß Anspruch 7 zur Behandlung von Atemwegserkrankungen oder systemischen Erkrankungen.

15. Pulver gemäß einem der Ansprüche 1 bis 6 oder eine pharmazeutische Zusammensetzung gemäß Anspruch 7 zur Behandlung von Atemwegserkrankungen oder systemischen Erkrankungen gemäß Anspruch 14, wobei die Erkrankung ausgewählt ist aus der Gruppe bestehen aus Lungenkrebs, Lungenentzündung, cystische Fibrose, COPD (chronisch obstruktive Lungenerkrankung), Asthma, anti-inflammatorische Erkrankungen, virale Erkrankungen wie z.B. durch den respiratorisch-syncytial Virus (RSV).

16. Verwendung eines Pulvers gemäß der Ansprüche 1 bis 6 oder einer pharmazeutischen Zusammensetzung gemäß Anspruch 7 zur Herstellung eines Medikaments zur Behandlung von Atemwegserkrankungen.

17. Verwendung gemäß Anspruch 16 wobei die Erkrankung ausgewählt ist aus der Gruppe bestehen aus Lungenkrebs, Lungenentzündung, cystische Fibrose, COPD (chronisch obstruktive Lungenerkrankung), Asthma, anti-inflammatorische Erkrankungen, virale Erkrankungen wie z.B. durch den respiratorisch-syncytial Virus (RSV).

## Claims

1. Powder comprising a protein and phenylalanine, **characterised in that** the powder comprises at least 30 % (w/w) phenylalanine, preferably at least 40 % (w/w) phenylalanine.

2. Powder according to claim 1, **characterised in that** the powder comprises at least one further excipient such as a sugar or a polyol.

3. Powder according to claim 1 or 2, **characterised in that** the powder is spraydried.

4. Powder according to one of claims 2 to 3, **characterised in that** the sugar is a non-reducing sugar selected from among a disaccharide and an oligosaccharide.

5. Powder according to one of claims 1 to 3, **characterised in that** the protein is an active substance, preferably a pharmaceutical active substance such as an antibody, an antibody fragment, a fusion protein with parts of antibodies or a conjugated antibody, a growth factor, a hormone or an enzyme.

6. Powder according to one of claims 1 to 5, **characterised in that** the mean aerodynamic particle size (MMAD = mass median aerodynamic diameter) of the powder particles is less than 10 µm, preferably less than 7.5 µm, more preferably in the range between 1-6 µm or 3-6 µm or 5-7 µm.

7. Pharmaceutical composition comprising a powder according to one of Claims 1 to 6.

8. Method of preparing a powder according to one of claims 1 to 6, **characterised in that**
a) a phenylalanine solution is prepared,
b) at least one protein and optionally at least one further excipient such as a sugar or a polyol are added,
c) the solution or suspension thus obtained is sprayed at an inflow temperature of preferably 90 - 200°C and an outflow temperature of preferably 40 - 150°C and
d) the particles formed are separated from the drying gas.

9. Method according to claim 8, **characterised in that** the protein is a pharmaceutical active substance.

10. Method according to claim 8 or 9 **characterised in that** the following additional steps are carried out between steps a) and b) :
- heating the phenylalanine solution, preferably to 80°C,
- cooling the phenylalanine solution to below the denaturing temperature of the particular protein to be added in each case, the cooling preferably being to ambient temperature.

11. Method according to claim 8 to 10, **characterised in that** the solution or suspension is sprayed in step c) by means of at least one pressure nozzle or at least one rotary evaporator or at least one venturi nozzle or at least one ultrasound nebuliser or at least one two-substance nozzle.

12. Method according to claim 8 to 11, **characterised in that** the separation of the particles in step d) is carried out using at least one particle separator, preferably at least one cyclone.

13. Powder according to Claims 1 to 6 or a pharmaceutical composition according to claim 7 for use as medicaments.

14. Powder according to one of Claims 1 to 6 or a pharmaceutical composition according to claim 7 for treating respiratory complaints or systemic diseases.

15. Powder according to one of Claims 1 to 6 or a pharmaceutical composition according to claim 7 for treating respiratory complaints or systemic diseases according to claim 14, the disease being selected from among lung cancer, inflammation of the lung, cystic fibrosis, COPD (chronic obstructive pulmonary disease), asthma, anti-inflammatory diseases, viral diseases e.g. caused by respiratory-syncytial virus (RSV) .

16. Use of a powder according to one of Claims 1 to 6 or a pharmaceutical composition according to claim 7 for preparing a medicament for the treatment of respiratory complaints.

17. Use according to claim 16, wherein the disease is selected from among lung cancer, inflammation of the lung, cystic fibrosis, COPD (chronic obstructive pulmonary disease), asthma, anti-inflammatory diseases, viral diseases e.g. caused by respiratory-syncytial virus (RSV) .

## Revendications

1. Poudre contenant une protéine et de la phénylalanine **caractérisée en ce que** la poudre contient au moins 30 % (w/w) de phénylalanine, de préférence au moins 40 % (w/w) de phénylalanine.

2. Poudre selon la revendication 1 **caractérisée en ce que** la poudre contient au moins un autre adjuvant comme un sucre ou un polyol.

3. Poudre selon la revendication 1 ou 2 **caractérisée en ce que** la poudre est séchée par pulvérisation.

4. Poudre selon l'une des revendications 2 à 3 **caractérisée en ce que** le sucre est un sucre non réducteur choisi dans le groupe consistant en un disaccharide et un oligosaccharide.

5. Poudre selon l'une des revendications 1 à 3 **caractérisée en ce que** la protéine est un principe actif, de préférence un principe actif pharmaceutique comme un anticorps, un fragment d'anticorps, une protéine de fusion avec des parties d'anticorps ou un anticorps conjugué, un facteur de croissance, une hormone ou une enzyme.

6. Poudre selon l'une des revendications 1 à 5 **caractérisée en ce que** la granulométrie aérodynamique moyenne (MMAD = mass median aerodynamic diameter) des particules de poudre est inférieure à 10 µm, de préférence inférieure à 7,5 µm, de préférence encore située dans le domaine de 1-6 µm ou 3-6 µm ou 5-7 µm

7. Composition pharmaceutique contenant une poudre selon l'une des revendications 1 à 6.

8. Procédé pour produire une poudre selon l'une des revendications 1 à 6 **caractérisé en ce que**
a) une solution de phénylalanine est produite,
b) au moins une protéine et éventuellement au moins un autre adjuvant comme un sucre ou un polyol sont ajoutés,
c) la solution ou suspension ainsi obtenue est pulvérisée à une température d'entrée de préférence de 90 - 200°C et à une température de sortie de préférence de 40 - 150°C et
d) les particules formées sont séparées du gaz de séchage.

9. Procédé selon la revendication 8 **caractérisé en ce que** la protéine est un principe actif pharmaceutique.

10. Procédé selon la revendication 8 ou 9 **caractérisé en ce que** les étapes supplémentaires suivantes sont réalisées entre les étapes a) et b)
- un chauffage de la solution de phénylalanine, de préférence à 80°C,
- un refroidissement de la solution de phénylalanine jusqu'en dessous de la température de dénaturation de la protéine à ajouter dans chaque cas, le refroidissement ayant lieu de préférence jusqu'à la température ambiante.

11. Procédé selon les revendications 8 à 10 **caractérisé en ce que** la solution ou suspension dans l'étape c) est pulvérisée au moyen d'au moins une buse à pression ou d'au moins un pulvérisateur rotatif ou d'au moins un tube de Venturi ou d'au moins un nébuliseur à ultrasons ou d'au moins une buse à deux substances.

12. Procédé selon les revendications 8 à 11 **caractérisé en ce que** la séparation des particules dans l'étape d) a lieu au moyen d'au moins un séparateur de particules, de préférence au moyen d'au moins un cyclone.

13. Poudre selon les revendications 1 à 6 ou composition pharmaceutique selon la revendication 7 destinée à être utilisée comme médicament.

14. Poudre selon l'une des revendications 1 à 6 ou composition pharmaceutique selon la revendication 7 destinée au traitement des maladies des voies respiratoires ou des maladies systémiques.

15. Poudre selon l'une des revendications 1 à 6 ou composition pharmaceutique selon la revendication 7 destinée au traitement des maladies des voies respiratoires ou des maladies systémiques selon la revendication 14 où la maladie est choisie dans le groupe consistant en le cancer du poumon, l'inflammation du poumon, la mucoviscidose, la COPD (bronchopneumopathie chronique obstructive), l'asthme, les maladies anti-inflammatoires, les maladies virales dues par exemple au virus respiratoire syncytial (VRS).

16. Utilisation d'une poudre selon les revendications 1 à 6 ou d'une composition pharmaceutique selon la revendication 7 pour la production d'un médicament pour le traitement des maladies des voies respiratoires.

17. Utilisation selon la revendication 16 où la maladie est choisie dans le groupe consistant en le cancer du poumon, l'inflammation du poumon, la mucoviscidose, la COPD (bronchopneumopathie chronique obstructive), l'asthme, les maladies anti-inflammatoires, les maladies virales dues par exemple au virus respiratoire syncytial (VRS).
